Europäisches Patentamt

European Patent Office

Office européen des brevets

(19)

(11) **EP 1 380 655 A2**

## (12) EUROPEAN PATENT APPLICATION

(43) Date of publication:
**14.01.2004 Bulletin 2004/03**

(51) Int Cl.[7]: **C12Q 1/68**

(21) Application number: **03011201.5**

(22) Date of filing: **16.05.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **17.05.2002 US 150792**

(71) Applicant: **MAYO FOUNDATION FOR MEDICAL
EDUCATION AND RESEARCH
Rochester, MN 55905 (US)**

(72) Inventors:
• **Cockerill III, Franklin R.
Rochester, Minnesota 55905 (US)**

• **Rosenblatt, Jon E.
Rochester, Minnesota 55906 (US)**
• **Sloan, Lynne M.
Rochester, Minnesota 55902 (US)**
• **Uhl, James R.
Rochester, Minnesota 55902 (US)**

(74) Representative: **Gassner, Wolfgang, Dr.
Patentanwalt,
Nägelsbachstrasse 49a
91052 Erlangen (DE)**

## (54) Detection of Shiga toxin- or Shiga-like toxin-producing organisms

(57)    The invention provides methods to detect Shiga toxin- or Shiga-like toxin-producing organisms, particularly Shiga-like toxin-producing *E. coli* organisms, in biological samples using real-time PCR. Primers and probes for the detection of Shiga toxin- or Shiga-like toxin-producing organisms are provided by the invention. Articles of manufacture containing such primers and probes for detecting Shiga toxin- or Shiga-like toxin-producing organisms are further provided by the invention.

**EP 1 380 655 A2**

**Description**

**TECHNICAL FIELD**

This invention relates to bacterial diagnostics, and more particularly to detection of Shiga toxin- or Shiga-like toxin-producing organisms, specifically Shiga-like toxin-producing *Escherichia coli* organisms.

**BACKGROUND**

Bacteria that belong to the four species of the genus Shigella cause a dysenteric syndrome by means of their unique capacity to invade the human colonic mucosa. Current epidemics of *Shigella dysenteriae* serotype 1 strains are particularly serious and are characterized by high mortality rates. Many of the isolates are resistant to many of the antibiotics currently in use in these countries. Biologically, the Shiga toxin produced by *Shigella* contributes to pathogenesis by directly damaging vascular endothelial cells, thereby disrupting the homeostatic properties of these cells. For a review relating to *Shigella* and Shiga toxin, see, for example, Shears, 1996, Ann. Trop. Med. Parasitol., 90:105-14.
Shiga-like toxins are a major virulence factor for disease-causing *E. coli.* Shiga-like toxin-producing *E. coli* organisms are responsible for outbreaks of gastrointestinal disease in humans, especially in the young and old. Shiga-like toxin-producing *E. coli* organisms also are associated with severe forms of disease such as hemorrhagic colitis (HC) and hemolytic uremic syndrome (HUS). Because the mortality rate of HUS is 5-10%, rapid detection is important for effective treatment. For reviews relating to *E. coli* Shiga-like toxin, see Paton & Paton (1998, *Clin. Microbiol. Rev.*, 11(3):450-79), Karch et al. (1999, *Diag. Microbiol. Infect. Dis*., 34(3):229-43) and Acheson et al. (2000, In *Bacterial Toxins: Methods and Protocols,* Vol. 45, Humana Press Inc., Totowa, pp. 41-63).

**SUMMARY**

The invention provides for methods of identifying Shiga toxin- or Shiga-like toxin-producing organisms in a biological sample. Primers and probes for detecting nucleic acids encoding Shiga toxin or Shiga-like toxin are provided by the invention, as are kits containing such primers and probes. Methods of the invention can be used to rapidly identify nucleic acids encoding Shiga toxin or Shiga-like toxin from specimens for diagnosis of illnesses caused by Shiga toxin or Shiga-like toxin. Using specific primers and probes, the methods include amplifying and monitoring the development of specific amplification products using real-time PCR.
In one aspect of the invention, there is provided a method for detecting the presence or absence of one or more Shiga toxin- or Shiga-like toxin-producing organisms in a biological sample from an individual. The method to detect Shiga toxin- or Shiga toxin-producing organisms includes performing at least one cycling step, which includes an amplifying step and a hybridizing step. The amplifying step includes contacting the sample with a pair of *stx1* primers to produce an amplification product if a nucleic acid molecule encoding a Shiga toxin- or Shiga-like toxin is present in the sample. The hybridizing step includes contacting the sample with a pair of *stx1* probes. Generally, the members of the pair of *stx1* probes hybridize to the amplification product within no more than five nucleotides of each other. A first *stx1* probe of the pair of *stx1* probes is typically labeled with a donor fluorescent moiety and a second *stx1* probe of the pair of *stx1* probes is typically labeled with a corresponding acceptor fluorescent moiety. The method further includes detecting the presence or absence of fluorescence resonance energy transfer (FRET) between the donor fluorescent moiety of the first *stx1* probe and the acceptor fluorescent moiety of the second *stx1* probe. The presence of FRET is usually indicative of the presence of one or more Shiga toxin- or Shiga-like toxin-producing organisms in the biological sample, while the absence of FRET is usually indicative of the absence of a Shiga toxin- or Shiga-like toxin-producing organism in the biological sample. In certain cases, the organism is *E. coli* and the toxin is a Shiga-like toxin.
Alternatively or additionally, the amplifying step can include contacting the sample with a pair of *stx2* primers to produce a *stx2* amplification product if an *E. coli* Shiga-like toxin *stx2* nucleic acid molecule is present in the sample. The hybridizing step includes contacting the sample with a pair of *stx2* probes. Generally, the members of the pair of *stx2* probes hybridize to the amplification product within no more than five nucleotides of each other. A first *stx2* probe of the pair of *stx2* probes is typically labeled with a donor fluorescent moiety and a second *stx2* probe of the pair of *stx2* probes is typically labeled with a corresponding acceptor fluorescent moiety. The method further includes detecting the presence or absence of FRET between the donor fluorescent moiety of the first *stx2* probe and the acceptor fluorescent moiety of the second *stx2* probe. The presence of FRET usually indicates the presence of one or more Shiga-like toxin-producing *E. coli* organisms in the biological sample, and the absence of FRET usually indicates the absence of a Shiga-like toxin-producing *E. coli* organism in the biological sample.
A pair of *stx1* primers generally includes a first *stx1* primer and a second *stx1* primer. The first *stx1* primer can include the sequence 5'-CAA GAG CGA TGT TAC GGT-3' (SEQ ID NO:1), and the second *stx1* primer can include the sequence 5'-AAT TCT TCC TAC ACGAAC AGA-3' (SEQ ID NO:2). A first *stx1* probe can include the sequence 5'-CTG GGG AAG GTT GAG TAG CG-3' (SEQ ID NO:3), and the second *stx1* probe can include the sequence 5'-CCT GCC TGA CTA TCA TGG ACA-3' (SEQ ID NO:4).

A pair of *stx2* primers generally includes a first *stx2* primer and a second *stx2* primer. A first *stx2* primer can include the sequence 5'-GGG ACC ACA TCG GTG T-3' (SEQ ID NO:5), and the second *stx2* primer can include the sequence 5'-CGG GCA CTG ATA TAT GTG TAA-3' (SEQ ID NO:6). A first *stx2* probe can include the sequence 5'-CTG TGG ATA TAC GAG GGC TTG ATG TC-3' (SEQ ID NO:7), and the second *stx2* probe can include the sequence 5'-ATC AGG CGC GTT TTG ACC ATC T-3' (SEQ ID NO:8).

In some aspects, one of the *stx1* or *stx2* primers can be labeled with a fluorescent moiety (either a donor or acceptor, as appropriate) and can take the place of one of the stx 1 or *stx2* probes, respectively.

The members of the pair of *stx1* probes can hybridize within no more than two nucleotides of each other, or can hybridize within no more than one nucleotide of each other. A representative donor fluorescent moiety is fluorescein, and corresponding acceptor fluorescent moieties include LC-Red 640, LC-Red 705, Cy5, and Cy5.5. Additional corresponding donor and acceptor fluorescent moieties are known in the art.

In one aspect, the detecting step includes exciting the biological sample at a wavelength absorbed by the donor fluorescent moiety and visualizing and/or measuring the wavelength emitted by the acceptor fluorescent moiety (*i.e.*, visualizing and/or measuring FRET). In another aspect, the detecting comprises quantitating the FRET. In yet another aspect, the detecting step can be performed after each cycling step (*i.e.*, in real-time).

Generally, the presence of FRET within 50 cycles (e.g., 20, 25, 30, 35, 40, or 45 cycles) indicates the presence of a Shiga toxin- or Shiga-like toxin-producing organism in the individual. In addition, determining the melting temperature between one or both of the *stx1* probe(s) and the amplification product, wherein the melting temperature confirms the presence or the absence of a Shiga toxin- or Shiga-like toxin-producing organism, while determining the melting temperature between one or both of the *stx2* probe(s) and the *stx2* amplification product confirms the presence or the absence of a Shiga-like toxin-producing *E. coli* organism.

Representative biological samples include stool samples and body fluids. The above-described methods can further include preventing amplification of a contaminant nucleic acid. Preventing amplification of a contaminant nucleic acid can include performing the amplifying step in the presence of uracil and treating the biological sample with uracil-DNA glycosylase prior to amplifying.

In addition, the cycling step can be performed on a control sample. A control sample can include a nucleic acid molecule encoding a Shiga toxin or Shiga-like toxin. Alternatively, a control sample can include a nucleic acid molecule other than a Shiga toxin or Shiga-like toxin nucleic acid molecule. Cycling steps can be performed on such a control sample using a pair of control primers and a pair of control probes. The control primers and the control probes are other than the *stx1* or *stx2* primers and probes. One or more amplifying steps can produce a control amplification product. Each of the control probes hybridizes to the control amplification product.

In another aspect of the invention, there are provided articles of manufacture, or kits. Kits of the invention can include a pair of *stx1* primers; a pair of *stx1* probes; and a donor fluorescent moiety and a corresponding fluorescent moiety. For example, a first *stx1* primer provided in a kit of the invention can include the sequence 5'-CAA GAG CGA TGT TAC GGT-3' (SEQ ID NO:1), and the second *stx1* primer can include the sequence 5'-AAT TCT TCC TAC ACG AAC AGA-3' (SEQ ID NO:2). A first *stx1* probe can include the sequence 5'-CTG GGG AAG GTT GAG TAG CG-3' (SEQ ID NO:3), and the second *stx1* probe can include the sequence 5'-CCT GCC TGA CTA TCA TGG ACA-3' (SEQ ID NO:4).

Articles of manufacture of the invention can further or alternatively include a pair of *stx2* primers; a pair of *stx2* probes; and a donor fluorescent moiety and a corresponding fluorescent moiety. For example, the first *stx2* primer provided in a kit of the invention can include the sequence 5'-GGG ACC ACA TCG GTG T-3' (SEQ ID NO:5), and the second *stx2* primer can include the sequence 5'-CGG GCA CTG ATA TAT GTG TAA-3' (SEQ ID NO:6). The first *stx2* probe provided in a kit of the invention can include the sequence 5'-CTG TGG ATA TAC GAG GGC TTG ATG TC-3' (SEQ ID NO:7), and the second *stx2* probe can include the sequence 5'-ATC AGG CGC GTT TTG ACC ATC T-3' (SEQ ID NO:8).

Articles of the invention can include fluorophoric moieties for labeling the probes, or probes already labeled with donor and corresponding acceptor fluorescent moieties. The article of manufacture can also include a package label or package insert having instructions thereon for using the pair of *stx1* primers and the pair of *stx1* probes to detect the presence or absence of one or more Shiga toxin- or Shiga-like toxin-producing organisms in a biological sample, or for using the pair of *stx2* primers and the pair of *stx2* probes to detect the presence or absence of one or more Shiga-like toxin-producing *E. coli* organisms in a biological sample.

In yet another aspect of the invention, there is provided a method for detecting the presence or absence of one or more Shiga toxin- or Shiga-like toxin-producing organisms in a biological sample from an individual. Such a method includes performing at least one cycling step. A cycling step can include an amplifying step and a hybridizing step. Generally, an amplifying step includes contacting the sample with a pair of *stx1* primers to produce an amplification product if a nucleic acid molecule encoding Shiga toxin or Shiga-like toxin is present in the sample. Generally, a hybridizing step includes contacting the sample with a *stx1* probe. Such a *stx1* probe is usually labeled with a donor fluorescent moiety and a corresponding acceptor fluorescent moiety. The method further includes detecting the presence or absence of FRET between the donor fluorescent moiety and the acceptor fluorescent moiety of the *stx1* probe. The presence or absence of FRET is indicative of the presence or absence of one or more Shiga toxin- or Shiga-like toxin-producing organisms in the sample.

In one aspect, amplification can employ a polymerase enzyme having 5' to 3' exonuclease activity. Thus, the donor and acceptor fluorescent moieties would be within no more than 5 nucleotides of each other along the length of the probe. In another aspect, the *stx1* probe includes a nucleic acid sequence that permits secondary structure formation. Such secondary structure formation generally results in spatial proximity between the donor and the acceptor fluorescent moiety. According to this method, the acceptor fluorescent moiety is a quencher.

In another aspect of the invention, there is provided a method for detecting the presence or absence of one or more Shiga toxin- or Shiga-like toxin-producing organisms in a biological sample from an individual. Such a method includes performing at least one cycling step. A cycling step can include an amplifying step and a dye-binding step. An amplifying step generally includes contacting the sample with a pair of *stx1* primers to produce an amplification product if a nucleic acid molecule encoding Shiga toxin or Shiga-like toxin is present in the sample. A dye-binding step generally includes contacting the amplification product with a double-stranded nucleic acid binding dye. The method further includes detecting the presence or absence of binding of the double-stranded nucleic acid binding dye to the amplification product. According to the invention, the presence of binding is typically indicative of the presence of one or more Shiga toxin- or Shiga-like toxin-producing organisms in the sample, and the absence of binding is typically indicative of the absence of a Shiga toxin- or Shiga-like toxin-producing organism in the sample. Such a method can further include the steps of determining the melting temperature between the amplification product and the double-stranded nucleic acid binding dye. Generally, the melting temperature confirms the presence or absence of the Shiga toxin- or Shiga-like toxin-producing organism. Representative double-stranded nucleic acid binding dye include SYBRGreenl®, SYBRGold®, and ethidium bromide.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting. All publications, patent applications, patents, and other references mentioned herein are incorporated by reference in their entirety. In case of conflict, the present specification, including definitions, will control.

The details of one or more embodiments of the invention are set forth in the accompanying drawings and the description below. Other features, objects, and advantages of the invention will be apparent from the drawings and detailed description, and from the claims.

## DESCRIPTION OF THE DRAWINGS

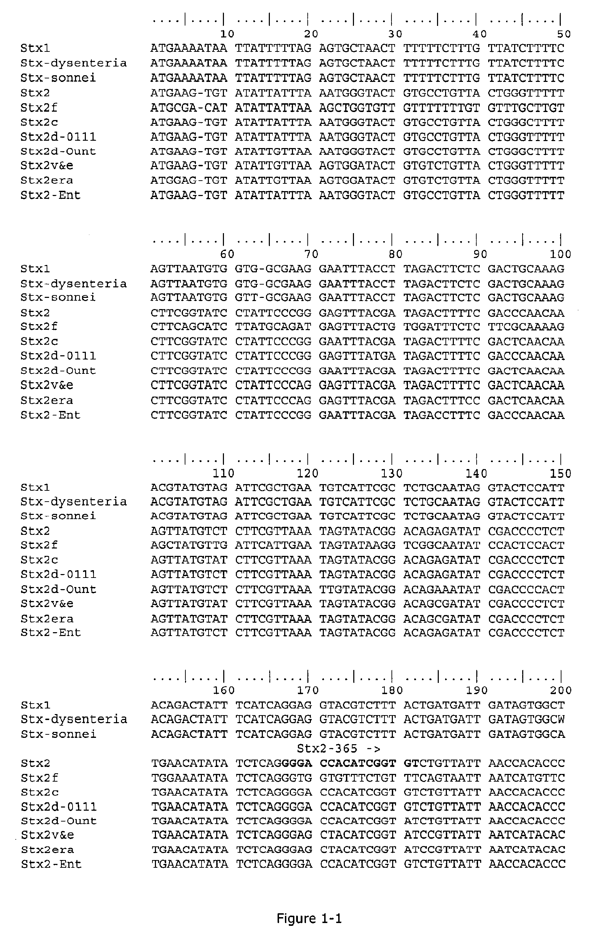

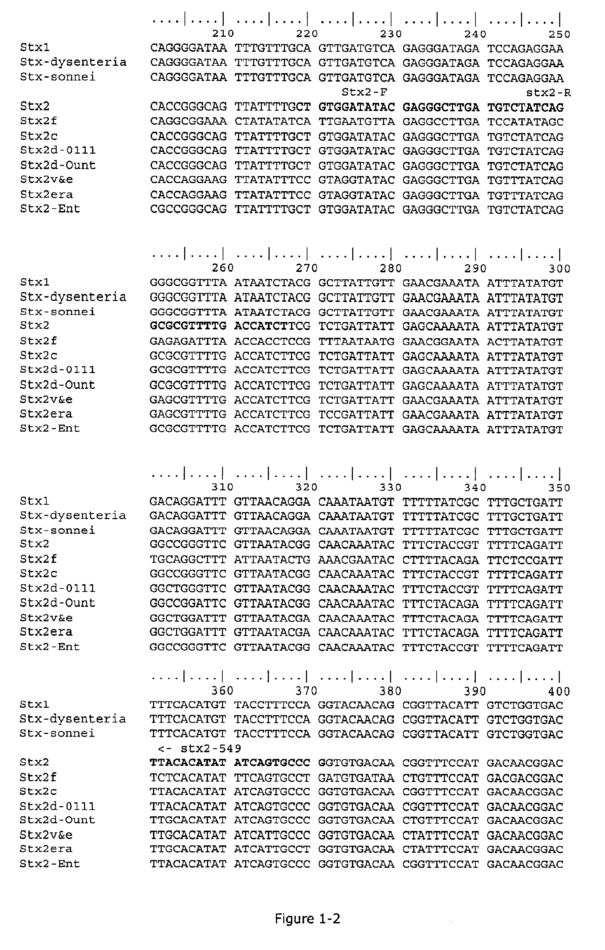

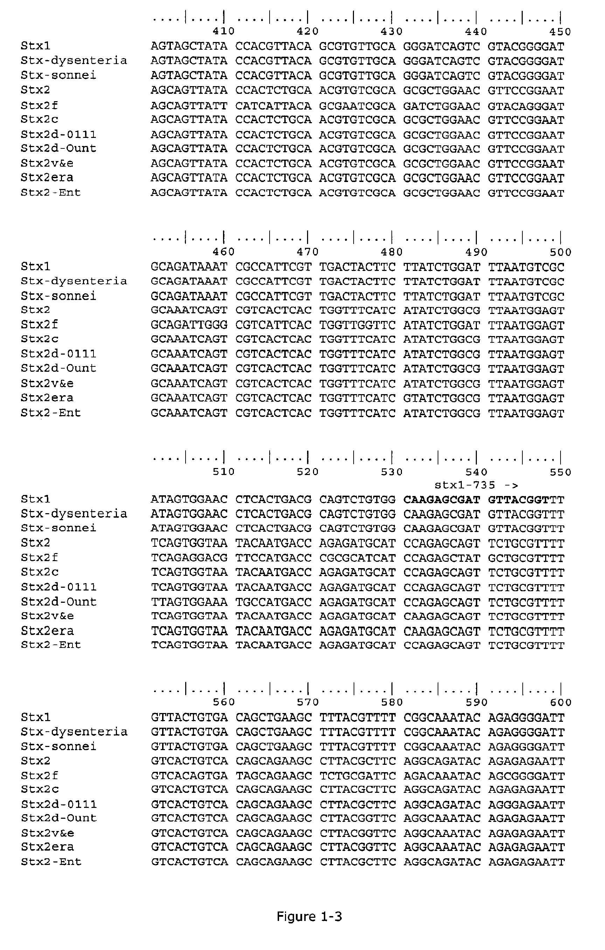

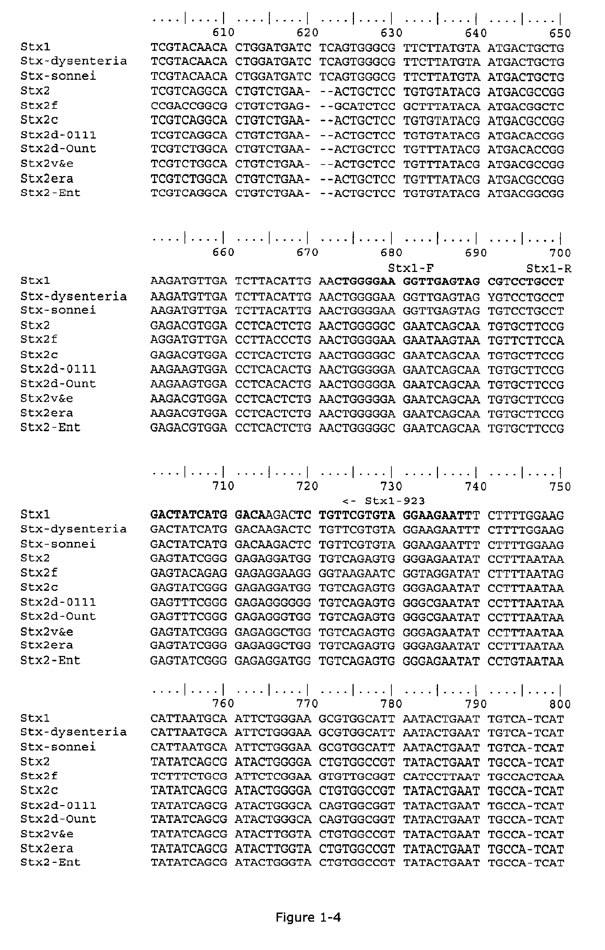

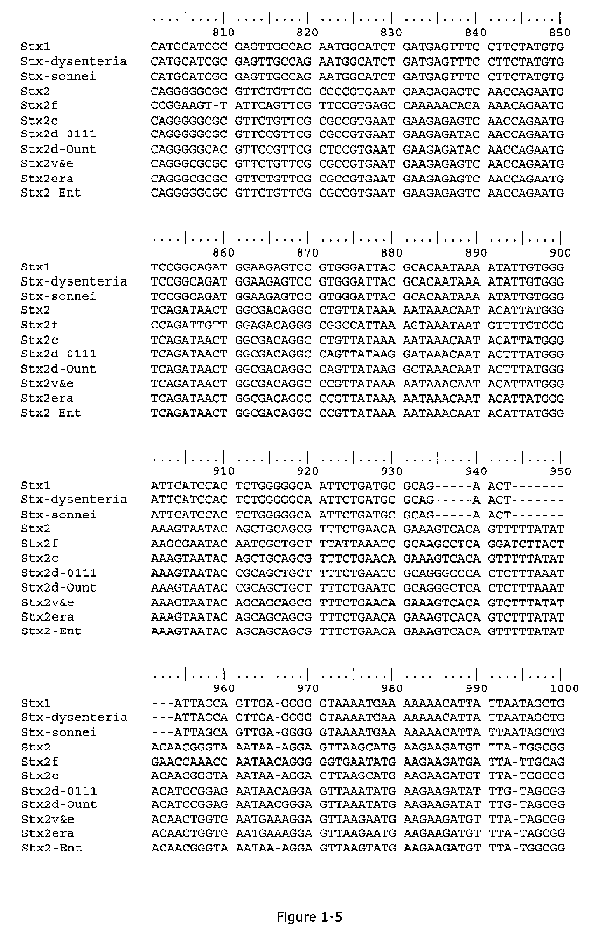

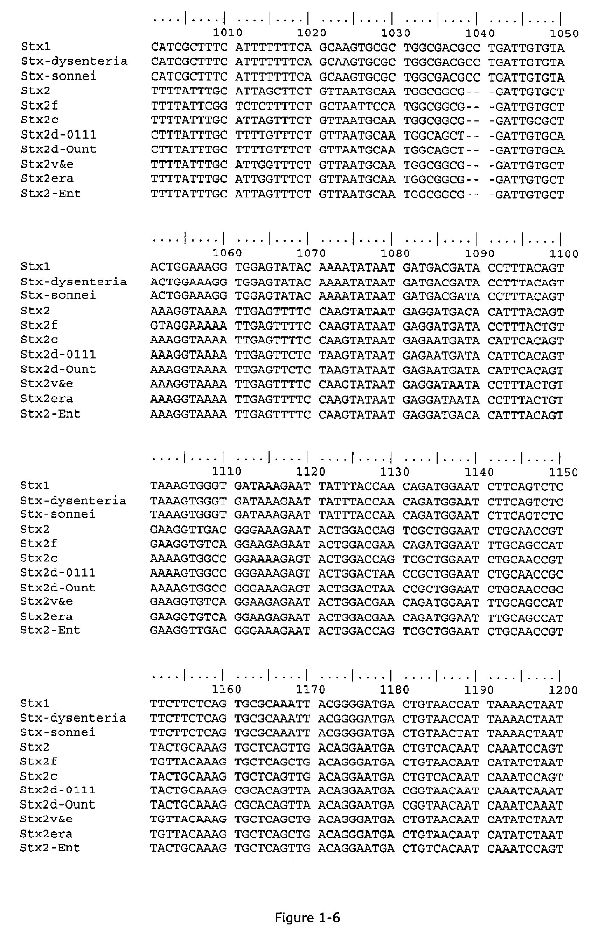

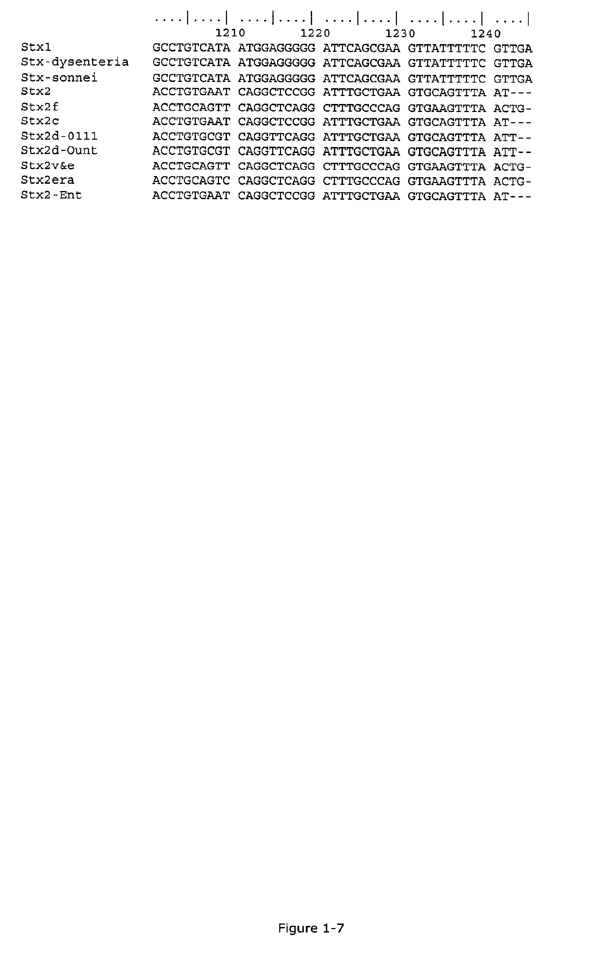

*Figure 1* is an alignment of nucleic acid sequences encoding Shiga-like toxins from several Shiga-like toxin-producing *E. coli* strains. The location of the primers and probes used herein for the real-time PCR assay are shown.

## DETAILED DESCRIPTION

A real-time PCR assay that is more sensitive than existing assays is described herein for detecting Shiga toxin- or Shiga-like toxin-producing organisms in a biological sample. Primers and probes for detecting nucleic acids encoding Shiga toxin or Shiga-like toxin are provided by the invention, as are articles of manufacture containing such primers and probes. The increased sensitivity of the real-time PCR assay for detecting nucleic acids encoding Shiga toxin or Shiga-like toxin compared to other methods, as well as the improved features of real-time PCR including sample containment and real-time detection of the amplified product, make feasible the implementation of this technology for routine clinical laboratory diagnosis of illnesses due to one or more Shiga toxins or Shiga-like toxins. In addition, the real-time PCR described herein can be used to detect and differentiate *Shigella* from *E. coli* using *stx1* and *stx2* primer and probe sets.

### *Escherichia coli* Shiga-like toxin

The Shiga-like toxin is composed of a toxin precursor, the A subunit, and a pentameric B subunit that binds to a cell surface receptor globotriaozylceramide. The A subunit is nicked by furin, a eukaryotic membrane-bound protease, thereby generating an active A1 fragment. The A1 fragment is very similar to ricin, a plant toxin. The mode of action of the Shiga-like toxin is inhibition of protein synthesis by modification of the ribosome.

The source of an *E. coli* Shiga-like toxin illness is usually from meat, especially cow, contaminated during processing. Another source is contaminated food from an infected human handler.

Some individuals infected with Shiga-like toxin-producing *E. coli* organisms are asymptomatic. For example, the incidence of asymptomatic carriers in Canadian dairy farm families can be as high as 6%. In addition, a study of 403 stool samples from children in central France detected Shiga-like toxin-producing *E. coli* in 11 children (2.7%) using conventional PCR. Using the same conventional PCR assay, 255 stool samples from children with diarrhea were tested and Shiga-like toxin-producing *E. coli* organisms were found in 8 samples (3.1%).

Shiga toxin and Shiga-like toxin nucleic acids and oligonucleotides

There are three related Shiga toxin or Shiga-like toxin genes reported in the literature; *stx, stx1* and *stx2*. The *stx* gene is found in *Shigella dysenteriae* and encodes "Shiga toxin", while *stx1* and *stx2* are found primarily in *E. coli* and encode "Shiga-like toxins". The nomenclature for Shiga-like toxins (also called Verotoxins) has been varied. For example, nucleic acids encoding Shiga-like toxin type 2 and Shiga-like toxin type 2 polypeptides have been designated: VT2, VT-2, VTII, VT-II, SLT2, SLT-2, SLTII, SLT-II, *stx2, stx-2,* and *stx$_2$*. Many variants of *stx2* have been found and have a letter attached to the number designation. A standard nomenclature was proposed by Calderwood et al. (1996, *ASM News*, 62:118-9) (Table 1).

Table 1

| Previous Nomenclature | Proposed Nomenclature | |
|---|---|---|
| | Gene | Protein |
| Shiga toxin (Stx) | *stx* | Stx |
| Shiga-like toxin-I (SLT-I) or Verotoxin 1 (VT1) | *stx1* | Stx1 |
| SLT-II or VT2 | *stx2* | Stx2 |
| SLT-lic or VT2c | *stx2c* | Stx2c |
| SLT-lie | *stx2e* | Stx2e |

Shiga toxin and Shiga-like toxin nomenclature is further complicated by the available sequence data. A search of the nucleic acid databases using BLAST software (http://www.ncbi.nlm.nih.gov) and an *stx* sequence as a query sequence indicates *stx* and *stx1* are identical except for three polymorphisms that are distributed between *S. dysenteriae* and *E. coli* isolates. Only one of the polymorphisms leads to an amino acid substitution. Therefore, methods that rely on *stx* or *stx1* nucleic acid sequences or antibodies to Stx or Stx1 cannot effectively discriminate between *S. dysenteriae* (*stx*) and *E. coli* (*stx1*) infections. In addition, gene variants of *stx2* are often associated with specific hosts (Table 2).

Table 2

| Shiga-like toxin | Hosts | Accession |
|---|---|---|
| *stx2* | Human, Cow | AF175707 AF298816 |
| *stx2c* | Human, Cow | M59432 |
| *stx2d* | Human, Sheep | L11078 |
| *stx2e* | Human, Porcine | M21534 |
| *stx2era* | Rabbit | U72191 |
| *stx2f* | Pigeon | AJ010730 |

A variety of bacteriophages carry the *stx1, stx2* or *stx2e* genes found in *E. coli.* The gene(s) occur occasionally in other *Enterobacteriaceae* such as *Citrobacter freundii, Aeromonas hydorophilia, A. caviae* and *Enterobacter cloacae.* The presence of *stx1, stx2,* and/or *stx2e* genes in *Enterobacteriaceae* are associated with disease but have not yet been associated with a bacteriophage. A bacteriophage source for the *stx* gene in *S. dysenteriae* type 1 also has not yet been demonstrated but there is evidence that the *stx* nucleic acid sequences are contiguous with phage sequences. Three *stx* genes (*stx1, stx2* and *stx2c*) have been found in one *E. coli* isolate and *E. coli* strains with both *stx1* and *stx2* are common. Therefore, infection of *E. coli* by bacteriophage carrying a particular Shiga-like toxin nucleic acid sequence does not confer immunity to infection by bacteriophage carrying other Shiga-like toxin nucleic acid sequences.

Transcription of *stx1* nucleic acid sequences is repressible by iron. The promoter region of *stx1* includes a recognition site for the *fur* gene product. Transcription of *stx2,* however, is not influenced by iron, osmolarity, pH, oxygen tension, acetates, or carbon source, but is activated by treatment with mitomycin. Mitomycin induces the lytic cycle of the *stx*-converting bacteriophages and is a positive regulator of the *stx2* promoter. This may explain why antibiotics seem to be beneficial for diseases caused by *stx* and detrimental for diseases cased by *stx2*.

The invention provides methods to detect nucleic acids encoding Shiga toxin or Shiga-like toxin by amplifying, for example, nucleic acid molecules corresponding to a portion of *stx1* or *stx2.* Nucleic acid molecules other than those exemplified herein *(e.g.,* other than *stx1* or *stx2)* also can be used to detect Shiga toxin- or Shiga-like toxin-producing organisms in a sample and are known to those of skill in the art. Nucleic acid sequences encoding *E. coli* Shiga-like toxin *stx1* or *stx2* have been described (see, for example, GenBank Accession Nos. AB048237, AJ413986, AB017524, or AB048240). See also GenBank Accession Nos. NC 002695 or AE005174 for the sequence of the *E. coli* O157:H7 genome. Shigella nucleic acids encoding Shiga toxin have been described (see, for example, GenBank Accession Nos. M19437, or AJ132761). Specifically, primers and probes to amplify and detect nucleic acids encoding *stx1* or *stx2* are provided by the invention.

Primers that amplify a nucleic acid molecule encoding Shiga toxin or Shiga-like toxin, *e.g*., nucleic acids encoding a portion of *stx1* or *stx2*, can be designed using, for example, a computer program such as OLIGO (Molecular Biology Insights Inc., Cascade, CO). Important features when designing oligonucleotides to be used as amplification primers include, but are not limited to, an appropriate size amplification product to facilitate detection (*e.g*., by electrophoresis), similar melting temperatures for the members of a pair of primers, and the length of each primer (*i.e*., the primers need to be long enough to anneal with sequence-specificity and to initiate synthesis but not so long that fidelity is reduced during oligonucleotide synthesis). Typically, oligonucleotide primers are 8 to 50 nucleotides in length (*e.g*., 10, 12, 14, 16, 18, 20, 22, 24, 26, 28, 30, 32, 34, 36, 38, 40, 42, 44, 46, 48, or 50 nucleotides in length). "*stx1* primers" or "*stx2* primers" as used herein refer to oligonucleotide primers that anneal to nucleic acid sequences encoding *stx1* or *stx2*, respectively, and initiate synthesis therefrom under appropriate conditions.

Designing oligonucleotides to be used as hybridization probes can be performed in a manner similar to the design of primers, although the members of a pair of probes preferably anneal to an amplification product within no more than 5 nucleotides of each other on the same strand such that fluorescent resonance energy transfer (FRET) can occur (e.g., within no more than 1, 2, 3, or 4 nucleotides of each other). This minimal degree of separation typically brings the respective fluorescent moieties into sufficient proximity such that FRET occurs. It is to be understood, however, that other separation distances (*e.g*., 6 or more nucleotides) are possible provided the fluorescent moieties are appropriately positioned relative to each other (for example, with a linker arm) such that FRET can occur. In addition, probes can be designed to hybridize to targets that contain a mutation or polymorphism, thereby allowing differential detection of Shiga toxin-producing *Shigella* organisms and Shiga-like toxin-producing *E. coli* organisms based on either absolute hybridization of different pairs of probes corresponding to each particular organism to be distinguished or differential melting temperatures between, for example, members of a pair of probes and each amplification product generated from a *Shigella* organism and an *E. coli* organism. For example, using appropriate probe pairs, *Shigella* organisms can be distinguished from *E. coli* organisms. As with oligonucleotide primers, oligonucleotide probes usually have similar melting temperatures, and the length of each probe must be sufficient for sequence-specific hybridization to occur but not so long that fidelity is reduced during synthesis. Oligonucleotide probes are 8 to 50 nucleotides in length (*e.g*., 10, 12, 14, 16, 18, 20, 22, 24, 26, 28, 30, 32, 34, 36, 38, 40, 42, 44, 46, 48, or 50 nucleotides in length). "*stx1* probes" or "*stx2* probes" as used herein refer to oligonucleotide probes that anneal to an *stx1* or *stx2* amplification product, respectively.

Constructs of the invention include vectors containing a nucleic acid molecule encoding a Shiga toxin or Shiga-like toxin, *e.g*., an *stx1* or *stx2* gene, or fragment thereof. Constructs can be used, for example, as a control template nucleic acid. Vectors suitable for use in the present invention are commercially available and/or produced by recombinant DNA technology methods routine in the art. A nucleic acid molecule encoding Shiga toxin or Shiga-like toxin, *e.g*., *stx1* or *stx2*, can be obtained, for example, by chemical synthesis, direct cloning from a *Shigella* or *E. coli* organism, or by PCR amplification. A Shiga toxin or Shiga-like toxin nucleic acid molecule or fragments thereof can be operably linked to a promoter or other regulatory element such as an enhancer sequence, a response element or an inducible element that modulates expression of the Shiga toxin or Shiga-like toxin nucleic acid molecule. As used herein, operably linking refers to connecting a promoter and/or other regulatory elements to a Shiga toxin or Shiga- like toxin nucleic acid molecule in such a way as to permit and/or regulate expression of the nucleic acid molecule. A promoter that does not normally direct expression of *stx, stx1*, or *stx2* can be used to direct transcription of an *stx, stx1*, or *stx2* nucleic acid molecule using, for example a viral polymerase, a bacterial polymerase, or a eukaryotic RNA polymerase. Alternatively, an *stx, stx1*, or *stx2* native promoter can be used to direct transcription of an *stx, stx1,* or *stx2* nucleic acid molecule using, for example, an *E. coli* RNA polymerase or a host RNA polymerase. In addition, operably linked can refer to an appropriate connection between an *stx, stx1*, or *stx2* promoter or other regulatory element to a heterologous coding sequence *(i.e.,* a non-*stx*, -*stx1*, or -*stx2* coding sequence, for example a reporter gene) in such a way as to permit expression of the heterologous coding sequence.

Constructs suitable for use in the methods of the invention typically include, in addition to an *stx, stx1*, or *stx2* nucleic acid molecule, sequences encoding a selectable marker (*e.g.*, an antibiotic resistance gene) for selecting desired constructs and/or transformants, and an origin of replication. The choice of vector systems usually depends upon several factors, including, but not limited to, the choice of host cells, replication efficiency, selectability, inducibility, and the ease of recovery.

Constructs of the invention containing an *stx, stx1*, or *stx2* nucleic acid molecule can be propagated in a host cell. As used herein, the term host cell is meant to include prokaryotes and eukaryotes such as yeast, plant and animal cells. Prokaryotic hosts can include *E. coli, Salmonella typhimurium, Serratia marcescens* and *Bacillus subtilis.* Eukaryotic hosts include yeasts such as *S. cerevisiae, S. pombe,* and *Pichia pastoris*, mammalian cells such as COS cells or Chinese hamster ovary (CHO) cells, insect cells, and plant cells such as *Arabidopsis thaliana* and *Nicotiana tabacum*. A construct of the invention can be introduced into a host cell using any of the techniques commonly known to those of ordinary skill in the art. For example, calcium phosphate precipitation, electroporation, heat shock, lipofection, microinjection, and viral-mediated nucleic acid transfer are common methods for introducing nucleic acids into host cells. In addition, naked DNA can be delivered directly to cells (see, *e.g.,* U.S. Patent Nos. 5,580,859 and 5,589,466).

Polymerase chain reaction (PCR)

U.S. Patent Nos. 4,683,202, 4,683,195, 4,800,159, and 4,965,188 disclose conventional PCR techniques. PCR typically employs two oligonucleotide primers that bind to a selected nucleic acid template (*e.g*., DNA or RNA). Primers useful in the present invention include oligonucleotides capable of acting as a point of initiation of nucleic acid synthesis within a Shiga-like toxin nucleic acid sequence. A primer can be purified from a restriction digest by conventional methods, or it can be produced synthetically. A primer is preferably single-stranded for maximum efficiency in amplification, but a primer can be double-stranded. Double-stranded primers are first denatured, *i.e.*, treated to separate the strands. One method of denaturing double-stranded nucleic acids is by heating.

The term "thermostable polymerase" refers to a polymerase enzyme that is heat stable, *i.e.*, the enzyme catalyzes the formation of primer extension products complementary to a template and does not irreversibly denature when subjected to the elevated temperatures for the time necessary to effect denaturation of double-stranded template nucleic acids. Generally, the synthesis is initiated at the 3' end of each primer and proceeds in the 5' to 3' direction along the template strand. Thermostable polymerases have been isolated from *Thermus flavus, T. ruber, T. thermophilus, T. aquaticus, T. lacteus, T. rubens, Bacillus stearothermophilus*, and *Methanothermus fervidus.* Nonetheless, polymerases that are not thermostable also can be employed in PCR provided the enzyme is replenished.

If the nucleic acid template is double-stranded, it is necessary to separate the two strands before it can be used as a template in PCR. Strand separation can be accomplished by any suitable denaturing method including physical, chemical or enzymatic means. One method of separating the nucleic acid strands involves heating the nucleic acid until it is predominately denatured (*e.g.*, greater than 50%, 60%, 70%, 80%, 90% or 95% denatured). The heating conditions necessary for denaturing template nucleic acid will depend, *e.g*., on the buffer salt concentration and the length and nucleotide composition of the nucleic acids being denatured, but typically range from about 90°C to about 105°C for a time depending on features of the reaction such as temperature and the nucleic acid length. Denaturation is typically performed for about 0 sec to 4 min.

If the double-stranded nucleic acid is denatured by heat, the reaction mixture is allowed to cool to a temperature that promotes annealing of each primer to its target sequence on the Shiga-like toxin nucleic acid. The temperature for annealing is usually from about 35°C to about 65°C. The reaction mixture is then adjusted to a temperature at which the activity of the polymerase is promoted or optimized, *e.g.*, a temperature sufficient for extension to occur from the annealed primer to generate products complementary to the template nucleic acid. The temperature should be sufficient to synthesize an extension product from each primer that is annealed to a nucleic acid template, but should not be so high as to denature an extension product from its complementary template. The temperature generally ranges from about 40° to 80°C.

PCR assays can employ nucleic acid template such as DNA or RNA, including messenger RNA (mRNA). The template nucleic acid need not be purified; it may be a minor fraction of a complex mixture, such as Shiga toxin and/or Shiga-like toxin nucleic acid contained in human cells. DNA or RNA may be extracted from any biological sample such as stool or body fluids (*e.g.*, cerebrospinal fluid (CSF), blood, or urine) by routine techniques such as those described in *Diagnostic Molecular Microbiology: Principles and Applications* (Persing et al. (eds), 1993, American Society for Microbiology, Washington D.C). Stool samples, however, are the preferred biological sample for detecting Shiga-like toxin-producing *E. coli* infections. *stx, stx1*, or *stx2* nucleic acids to be used as controls can be obtained from any number of sources, such as plasmids, or natural sources including bacteria, yeast, viruses, organelles, or higher organisms such as plants or animals.

The oligonucleotide primers are combined with other PCR reagents under reaction conditions that induce primer extension. For example, chain extension reactions generally include 50 mM KCl, 10 mM Tris-HCl (pH 8.3), 1.5 mM MgCl$_2$, 0.001% (w/v) gelatin, 0.5-1.0 Tg denatured template DNA, 50 pmoles of each oligonucleotide primer, 2.5 U of Taq polymerase, and 10% DMSO. The reactions usually contain 150 to 320 TM each of dATP, dCTP, dTTP, dGTP, or one or more analogs thereof. In certain circumstances, 300 to 640 μM dUTP can be substituted for dTTP in the reaction.

The newly synthesized strands form a double-stranded molecule that can be used in the succeeding steps of the reaction. The steps of strand separation, annealing, and elongation can be repeated as often as needed to produce the desired quantity amplification products corresponding to the target Shiga toxin or Shiga-like toxin nucleic acid molecule. The limiting factors in the reaction are the amounts of primers, thermostable enzyme, and nucleoside triphosphates present in the reaction. The cycling steps (*i.e.*, amplification and hybridization) are preferably repeated at least once. For use in detection, the number of cycling steps will depend, *e.g.*, on the nature of the sample. If the sample is a complex mixture of nucleic acids, more cycling steps may be required to amplify the target sequence sufficient for detection. Generally, the cycling steps are repeated at least about 20 times, but may be repeated as many as 40, 60, or even 100 times.

Fluorescent resonance energy transfer (FRET)

FRET technology (see, for example, U.S. Patent Nos. 4,996,143, 5,565,322, 5,849,489, and 6,162,603) is based on the fact that when a donor and a corresponding acceptor fluorescent moiety are positioned within a certain distance of each other, energy transfer takes place between the two fluorescent moieties that can be visualized or otherwise detected and/or quantitated. As used herein, two oligonucleotide probes, each containing a fluorescent moiety, can hybridize to an amplification product at particular positions determined by the complementarity of the oligonucleotide probes to the Shiga-like toxin target nucleic acid sequence. Upon hybridization of the oligonucleotide probes to the amplification product at the appropriate positions, a FRET signal is generated.

Fluorescent analysis can be carried out using, for example, a photon counting epifluorescent microscope system (containing the appropriate dichroic mirror and filters for monitoring fluorescent emission at the particular range), a photon counting photomultiplier system or a fluorometer. Excitation to initiate energy transfer can be carried out with an argon ion laser, a high intensity mercury (Hg) arc lamp, a fiber optic light source, or other high intensity light source appropriately filtered for excitation in the desired range.

As used herein with respect to donor and corresponding acceptor fluorescent moieties, "corresponding" refers to an acceptor fluorescent moiety having an emission spectrum that overlaps the excitation spectrum of the donor fluorescent moiety. The wavelength maximum of the emission spectrum of the acceptor fluorescent moiety preferably should be at least 100 nm greater than the wavelength maximum of the excitation spectrum of the donor fluorescent moiety. Accordingly, efficient non-radiative energy transfer can be produced therebetween.

Fluorescent donor and corresponding acceptor moieties are generally chosen for (a) high efficiency Förster energy transfer; (b) a large final Stokes shift (>100 nm); (c) shift of the emission as far as possible into the red portion of the visible spectrum (>600 nm); and (d) shift of the emission to a higher wavelength than the Raman water fluorescent emission produced by excitation at the donor excitation wavelength. For example, a donor fluorescent moiety can be chosen that has its excitation maximum near a laser line (for example, Helium-Cadmium 442 nm or Argon 488 nm), a high extinction coefficient, a high quantum yield, and a good overlap of its fluorescent emission with the excitation spectrum of the corresponding acceptor fluorescent moiety. A corresponding acceptor fluorescent moiety can be chosen that has a high extinction coefficient, a high quantum yield, a good overlap of its excitation with the emission of the donor fluorescent moiety, and emission in the red part of the visible spectrum (>600 nm).

Representative donor fluorescent moieties that can be used with various acceptor fluorescent moieties in FRET technology include fluorescein, Lucifer Yellow, B-phycoerythrin, 9-acridineisothiocyanate, Lucifer Yellow VS, 4-acetamido-4'-isothiocyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC™-Red 640, LC™-Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of Lanthanide ions (*e.g.*, Europium, or Terbium). Donor and acceptor fluorescent moieties can be obtained, for example, from Molecular Probes (Junction City, OR) or Sigma Chemical Co. (St. Louis, MO).

The donor and acceptor fluorescent moieties can be attached to the appropriate probe oligonucleotide via a linker arm. The length of each linker arm can be important, as the linker arms will affect the distance between the donor and the acceptor fluorescent moieties. The length of a linker arm for the purpose of the present invention is the distance in Angstroms (Å) from the nucleotide base to the fluorescent moiety. In general, a linker arm is from about 10 to about 25 Å. The linker arm may be of the kind described in WO 84/03285. WO 84/03285 also discloses methods for attaching linker arms to particular nucleotide bases, and also for attaching fluorescent moieties to a linker arm.

An acceptor fluorescent moiety such as an LC™-Red 640-NHS-ester can be combined with C6-Phosphoramidites (available from ABI (Foster City, CA) or Glen Research (Sterling, VA)) to produce, for example, LC™-Red 640-Phosphoramidite. Frequently used linkers to couple a donor fluorescent moiety such as fluorescein to an oligonucleotide include thiourea linkers (FITC-derived, for example, fluorescein-CPG's from Glen Research or ChemGene (Ashland, MA)), amide-linkers (fluorescein-NHS-ester-derived, such as fluorescein-CPG from BioGenex (San Ramon, CA)), or 3'-amino-CPG's that require coupling of a fluorescein-NHS-ester after oligonucleotide synthesis.

Detection of Shiga toxin- or Shiya-like toxin-producing organisms

The first *E. coli* serotype to be associated with HUS was O157:H7. To date, more than 160 antigenic variants of *E. coli* have been identified as Shiga-like toxin-producing *E. coli* organisms. The 0157 serotype can be easily differentiated in the microbiology laboratory from normal *E. coli* bowel flora because of its inability or delayed ability to ferment sorbitol and because of the absence of β-glucuronidase. Using sorbitol-MacConkey agar culture (SMAC) or Rainbow Agar O157® (Biolog Inc, Hayward, CA) which both contain substrates for β-d-glucuronidase and β-galactosidase, this strain can be discriminated from normal *E. coli* flora using standard culture techniques. Unfortunately, non-0157:H7 Shiga-like toxin-producing *E. coli* organisms go unidentified in most microbiology laboratories because they cannot be detected using standard culture techniques. In addition, aberrant biochemical properties of some O157:H isolates make detection of this serotype problematic. A consequence of the easy culture detection of O157:H7 serotypes is that the antigenic prevalence data is strongly biased toward this single serotype and the true incidence of diarrhea due to Shiga-like toxin-producing *E. coli* organisms is unknown.

Vero cells are susceptible to Shiga-like toxins, and can be used to detect toxin directly from stool specimens. Besides the technical difficulties of maintaining a Vero cell line, the assay requires 48 to 96 hours and is not entirely specific for Shiga-like toxins. Specific anti-toxins are needed to confirm positive results and the anti-toxins are not commercially available. Recent modifications to the cell culture assay in which lactate dehydroginase production from the Vero cells exposed to Shiga-like toxin is detected in a colorimetric assay (see, for example, Roberts et al., 2001, J. Microbiol. Methods, 43:171) have made the assay more rapid, requiring only 12 to 16 hours.

There are two commercially available ELISA assays for Shiga toxin or Shiga-like toxin: Premier EHEC (Meridian Diagnostics Inc, Cincinnati, OH) and ProSpect Shiga Toxin *E. coli* (Alexon Trend Inc, Ramsey, MN). Based on experiments performed in the Mayo Medical Laboratories, the sensitivities of the two kits are similar. The Premier EHEC kit is able to detect *stx* directly from diluted or broth cultured stool specimens and was found to have a sensitivity of 89% to 91% when compared to culture. The Premier EHEC is unable to detect variant *stx2e* and may produce false positive reactions with *Ps. aeruginosa.*

A confounding problem with the detection of Shiga-like toxin in cultures by Vero cell assay or by ELISA is the loss of the phage targets from the *E. coli* isolate during subculture. Loss of phage targets can lead to a false negative result. A method for detecting Shiga-like toxin-producing *E. coli* organisms directly from stool specimens is required to avoid this problem.

Latex agglutination assays, for example, VEROTOX-F and VTEC-RPLA (Denka Seiken Co, Ltd, Tokyo Japan), can be used to detect *E. coli* isolates. The latex agglutination assays do not work well on stool samples, and has generated false positives with seven of eight negative stool samples and false negatives for five of eight positive stool samples.

Diagnosis using serological testing has not been successful because patients do not typically develop an antibody response to Shiga-like toxins.

An immunofluorescent assay (KPL Inc., Gaithersburg, MD) using fluorescein-labeled 0157-specific antibody was developed for the rapid visualization of organisms in stool samples by fluorescent microscopy. This assay can detect sorbitol-positive *E. coli* 0157 strains (in contrast to culture), but additional testing is required to differentiate non-toxogenic 0157 strains from toxogenic 0157 strains. In addition, the immunofluorescent assay does not detect non-0157 Shiga-like toxin-producing *E. coli* organisms.

PCR has been a popular option for detecting Shiga toxin- or Shiga-like toxin-producing organisms. PCR assays to detect Shiga-like toxin-producing *E. coli* organisms can be divided into three groups: those that detect both *stx1* and *stx2* using a single pair of primers, those that perform multiplex PCR of *stx1* and *stx2*, and those that use other gene targets. Since no gene target other than *stx1* and *stx2* correlates 100% with Shiga-like toxin-producing *E. coli* organisms, the PCR assays that use a non-*stx1* or -*stx2* gene target are no longer used for detection of *E. coli.* Fourteen of the various PCR assays from the literature have been compared and it was found that the variability observed in amplification of the *stx2* gene made detection of Shiga-like toxin-producing *E. coli* organisms unreliable with some systems. In addition, a problem noted in most of the published PCR reactions is the presence of inhibitors in stool specimens.

The invention provides methods for detecting the presence or absence of one or more Shiga toxin- or Shiga-like toxin-producing organisms in a biological sample from an individual. Methods provided by the invention avoid problems of sample contamination, false negatives and false positives. The methods include performing at least one cycling step that includes amplifying and hybridizing. An amplification step includes contacting the biological sample with a pair of *stx1* or *stx2* primers to produce an amplification product if a Shiga toxin or Shiga-like toxin nucleic acid molecule is present in the sample. Each of the *stx1* or *stx2* primers anneals to a target within or adjacent to a Shiga toxin or Shiga-like toxin nucleic acid molecule such that at least a portion of the amplification product contains nucleic acid sequence corresponding to the respective Shiga toxin or Shiga-like toxin nucleic acid, and, more importantly, such that the amplification product contains the nucleic acid sequences that are complementary to *stx1* or *stx2* probes. A hybridizing step includes contacting the sample with a pair of *stx1* or *stx2* probes. Generally, the members of the pair of *stx1* or *stx2* probes hybridize to the appropriate amplification product within no more than five nucleotides of each other. According to the invention, a first *stx1* or *stx2* probe of the pair of *stx1* or *stx2* probes, respectively, is labeled with a donor fluorescent moiety and a second *stx1* or *stx2* probe of the pair of *stx1* or *stx2* probes, respectively, is labeled with a corresponding acceptor fluorescent moiety. The method further includes detecting the presence or absence of FRET between the donor fluorescent moiety of the first *stx1* or *stx2* probe and the corresponding acceptor fluorescent moiety of the second *stx1* or *stx2* probe. Multiple cycling steps can be performed, preferably in a thermocycler. The above-described methods for detecting Shiga toxin- or Shiga-like toxin-producing organism in a biological sample using primers and probes directed toward *stx1* or *stx2* also can be performed using other Shiga toxin or Shiga-like toxin gene-specific primers and probes.

As used herein, "amplifying" refers to the process of synthesizing nucleic acid molecules that are complementary to one or both strands of a template nucleic acid (*e.g.*, Shiga toxin or Shiga-like toxin nucleic acid molecules). Amplifying a nucleic acid molecule typically includes denaturing the template nucleic acid, annealing primers to the template nucleic acid at a temperature that is below the melting temperatures of the primers, and enzymatically elongating from the primers to generate an amplification product. The denaturing, annealing and elongating steps each can be performed once. Generally, however, the denaturing, annealing and elongating steps are performed multiple times such that the amount of amplification product is increasing, oftentimes exponentially, although exponential amplification is not required by the present methods. Amplification typically requires the presence of deoxyribonucleoside triphosphates, a DNA polymerase enzyme (*e.g.*, Platinum® Taq) and an appropriate buffer and/or co-factors for optimal activity of the polymerase enzyme (*e.g.*, MgCl$_2$ and/or KCl).

If amplification of Shiga toxin or Shiga-like toxin nucleic acid occurs and an amplification product is produced, the step of hybridizing results in a detectable signal based upon FRET between the members of the pair of probes. As used herein, "hybridizing" refers to the annealing of probes to an amplification product. Hybridization conditions typically include a temperature that is below the melting temperature of the probes but that avoids non-specific hybridization of the probes.

Generally, the presence of FRET indicates the presence of one or more Shiga toxin- or Shiga-like toxin-producing organisms in the biological sample, and the absence of FRET indicates the absence of Shiga toxin- or Shiga-like toxin-producing organisms in the biological sample. Inadequate specimen collection, transportation delays, inappropriate transportation conditions, or use of certain collection swabs (e.g., calcium alginate or aluminum shaft) are all conditions that can affect the success and/or accuracy of the test result, however. Using the methods disclosed herein, detection of FRET within 40 cycling steps is indicative of a Shiga toxin- or Shiga-like toxin-producing organism.

Representative biological samples that can be used in practicing the methods of the invention include stool samples or body fluids. Biological sample collection and storage methods are known to those of skill in the art. Biological samples can be processed (*e.g.*, by standard nucleic acid extraction methods and/or using commercial kits) to release nucleic acid encoding Shiga toxin or Shiga-like toxin or, in some cases, the biological sample is contacted directly with the PCR reaction components and the appropriate oligonucleotides.

Melting curve analysis is an additional step that can be included in a cycling profile. Melting curve analysis is based on the fact that DNA melts at a characteristic temperature called the melting temperature (Tm), which is defined as the temperature at which half of the DNA duplexes have separated into single strands. The melting temperature of a DNA depends primarily upon its nucleotide composition. Thus, DNA molecules rich in G and C nucleotides have a higher Tm than those having an abundance of A and T nucleotides. By detecting the temperature at which signal is lost, the melting temperature of probes can be determined. Similarly, by detecting the temperature at which signal is generated, the annealing temperature of probes can be determined. The melting temperature(s) of the *stx1* or *stx2* probes from the respective amplification product, respectively, can confirm the presence of a Shiga toxin- or Shiga-like toxin-producing organism in the sample.

Within each thermocycler run, control samples can be cycled as well. Control nucleic acid template can be amplified from a positive control sample (*e.g.*, template other than *stx1* or *stx2*) using, for example, control primers and control probes. Positive control samples can also be used to amplify, for example, a plasmid construct containing Shiga toxin or Shiga-like toxin nucleic acid molecules. Such a plasmid control can be amplified internally (*e.g.*, within each biological sample) or in separate samples run side-by-side with the patients' samples. Each thermocycler run also should include a negative control that, for example, lacks Shiga toxin or Shiga-like toxin template nucleic acid. Such controls are indicators of the success or failure of the amplification, hybridization, and/or FRET reaction. Therefore, control reactions can readily determine, for example, the ability of primers to anneal with sequence-specificity and to initiate elongation, as well as the ability of probes to hybridize with sequence-specificity and for FRET to occur.

In an embodiment, the methods of the invention include steps to avoid contamination. For example, an enzymatic method utilizing uracil-DNA glycosylase is described in U.S. Patent Nos. 5,035,996, 5,683,896 and 5,945,313 to reduce or eliminate contamination between one thermocycler run and the next. In addition, standard laboratory containment practices and procedures are desirable when performing methods of the invention. Containment practices and procedures include, but are not limited to, separate work areas for different steps of a method, containment hoods, barrier filter pipette tips and dedicated air displacement pipettes. Consistent containment practices and procedures by personnel are desirable for accuracy in a diagnostic laboratory handling clinical samples.

Conventional PCR methods in conjunction with FRET technology can be used to practice the methods of the invention. In one embodiment, a LightCycler™ instrument is used. A detailed description of the LightCycler™ System and real-time and on-line monitoring of PCR can be found at http://biochem.roche.com/lightcycler. The following patent applications describe real-time PCR as used in the LightCycler™ technology: WO 97/46707, WO 97/46714 and WO 97/46712. The LightCycler™ instrument is a rapid thermocycler combined with a microvolume fluorometer utilizing high quality optics. This rapid thermocycling technique uses thin glass cuvettes as reaction vessels. Heating and cooling of the reaction chamber are controlled by alternating heated and ambient air. Due to the low mass of air and the high ratio of surface area to volume of the cuvettes, very rapid temperature exchange rates can be achieved within the LightCycler™ thermal chamber. Addition of selected fluorescent dyes to the reaction components allows the PCR to be monitored in real-time and on-line. Furthermore, the cuvettes serve as an optical element for signal collection (similar to glass fiber optics), concentrating the signal at the tip of the cuvettes. The effect is efficient illumination and fluorescent monitoring of microvolume samples.

The LightCycler™ carousel that houses the cuvettes can be removed from the instrument. Therefore, samples can be loaded outside of the instrument (in a PCR Clean Room, for example). In addition, this feature allows for the sample carousel to be easily cleaned and sterilized. The fluorometer, as part of the LightCycler™ apparatus, houses the light source. The emitted light is filtered and focused by an epi-illumination lens onto the top of the cuvettes. Fluorescent light emitted from the sample is then focused by the same lens, passed through a dichroic mirror, filtered appropriately, and focused onto data-collecting photohybrids. The optical unit currently available in the LightCycler™ instrument (Catalog No. 2 011 468) includes three band-pass filters (530 nm, 640 nm, and 710 nm), providing three-color detection and several fluorescence acquisition options. Data collection options include once per cycling step monitoring, fully continuous single-sample acquisition for melting curve analysis, continuous sampling (in which sampling frequency is dependent on sample number) and/or stepwise measurement of all samples after defined temperature interval.

The LightCycler™ can be operated using a PC workstation and can utilize a Windows NT operating system. Signals from the samples are obtained as the machine positions the capillaries sequentially over the optical unit. The software can display the fluorescence signals in real-time immediately after each measurement. Fluorescent acquisition time is 10-100 msec. After each cycling step, a quantitative display of fluorescence vs. cycle number can be continually updated for all samples. The data generated can be stored for further analysis.

A common FRET technology format utilizes two hybridization probes. Each probe can be labeled with a different fluorescent moiety and the two probes are generally designed to hybridize in close proximity to each other in a target DNA molecule (e.g., an amplification product). By way of example, a donor fluorescent moiety such as fluorescein can be excited at 470 nm by the light source of the LightCycler™ Instrument. During FRET, fluorescein transfers its energy to an acceptor fluorescent moiety such as LightCycler™-Red 640 (LC™-Red 640) or LightCycler™-Red 705 (LC™-Red 705). The acceptor fluorescent moiety then emits light of a longer wavelength (*e.g.*, 640 nm or 705 nm, respectively), which is detected by the optical detection system of the LightCycler™ instrument. Other donor and corresponding acceptor fluorescent moieties suitable for use in the invention are described above. Efficient FRET can only take place when the fluorescent moieties are in direct local proximity (for example, within 5 nucleotides of each other as described above) and when the emission spectrum of the donor fluorescent moiety overlaps with the absorption spectrum of the acceptor fluorescent moiety. The intensity of the emitted signal can be correlated with the number of original target DNA molecules (e.g., the number of Shiga toxin- or Shiga-like toxin-producing organisms).

Another FRET technology format utilizes TaqMan® technology to detect the presence or absence of an amplification product, and hence, the presence or absence of Shiga toxin- or Shiga-like toxin-producing organisms. TaqMan® technology utilizes one single-stranded hybridization probe labeled with two fluorescent moieties. When a first fluorescent moiety is excited with light of a suitable wavelength, the absorbed energy is transferred to a second fluorescent moiety according to the principles of FRET. The second fluorescent moiety is generally a quencher molecule. During the annealing step of the PCR reaction, the labeled hybridization probe binds to the target DNA *(i.e.,* the amplification product) and is degraded by the 5' to 3' exonuclease activity of the Taq polymerase during the subsequent elongation phase. As a result, the excited fluorescent moiety and the quencher moiety become spatially separated from one another. As a consequence, upon excitation of the first fluorescent moiety in the absence of the quencher, the fluorescence emission from the first fluorescent moiety can be detected. By way of example, an ABI PRISM® 7700 Sequence Detection System (Applied Biosystems, Foster City, CA) uses TaqMan® technology, and is suitable for performing the methods described herein for detecting Shiga toxin- or Shiga-like toxin-producing organisms. Information on PCR amplification and detection using an ABI PRISM® 770 system can be found at http://www.appliedbiosystems.com/products.

Yet another FRET technology format utilizes molecular beacon technology to detect the presence or absence of an amplification product, and hence, the presence or absence of a Shiga toxin- or Shiga-like toxin-producing organism. Molecular beacon technology uses a hybridization probe labeled with a donor fluorescent moiety and an acceptor fluorescent moiety. The acceptor fluorescent moiety is generally a quencher, and the fluorescent labels are typically located at each end of the probe. Molecular beacon technology uses a probe oligonucleotide having sequences that permit secondary structure formation (*e.g.*, a hairpin). As a result of secondary structure formation within the probe, both fluorescent moieties are in spatial proximity when the probe is in solution. After hybridization to the target nucleic acids (*i.e.*, the amplification products), the secondary structure of the probe is disrupted and the fluorescent moieties become separated from one another such that after excitation with light of a suitable wavelength, the emission of the first fluorescent moiety can be detected.

As an alternative to detection using FRET technology, an amplification product can be detected using a nucleic acid binding dye such as a fluorescent DNA binding dye (*e.g.*, SYBRGreenI® or SYBRGold® (Molecular Probes)). Upon interaction with the double-stranded nucleic acid, such nucleic acid binding dyes emit a fluorescence signal after excitation with light at a suitable wavelength. A nucleic acid binding dye such as a nucleic acid intercalating dye also can be used. When nucleic acid binding dyes are used, a melting curve analysis is usually performed for confirmation of the presence of the amplification product.

It is understood that the present invention is not limited by the configuration of one or more commercially available instruments.

Articles of manufacture

The invention further provides for articles of manufacture to detect Shiga toxin- or Shiga-like toxin-producing organisms. An article of manufacture according to the present invention can include primers and probes used to detect nucleic acids from Shiga toxin- or Shiga-like toxin-producing organisms, together with suitable packaging material. Representative primers and probes provided in a kit for detection of Shiga toxin or Shiga-like toxin can be complementary to Shiga toxin or Shiga-like toxin nucleic acid molecules. Methods of designing primers and probes are disclosed herein, and representative examples of primers and probes that amplify and hybridize to Shiga toxin or Shiga-like toxin nucleic acid molecules are provided.

Articles of manufacture of the invention also can include one or more fluorescent moieties for labeling the probes or, alternatively, the probes supplied with the kit can be labeled. For example, an article of manufacture may include a donor fluorescent moiety for labeling one of the *stx1* or *stx2* probes and a corresponding acceptor fluorescent moiety for labeling the other *stx1* or *stx2* probe, respectively. Examples of suitable FRET donor fluorescent moieties and corresponding acceptor fluorescent moieties are provided herein.

Articles of manufacture of the invention also can contain a package insert having instructions thereon for using pairs of *stx1* or *stx2* primers and *stx1* or *stx2* probes to detect Shiga toxin- or Shiga-like toxin-producing organisms in a biological sample. Articles of manufacture may additionally include reagents for carrying out the methods disclosed herein (e.g., buffers, polymerase enzymes, co-factors, or agents to prevent contamination). Such reagents may be specific for one of the commercially available instruments described herein.

The invention will be further described in the following examples, which do not limit the scope of the invention described in the claims.

**EXAMPLES**

Example 1—Sample Preparation

*STEC Samples - ProSpecT® Shiga Toxin E. coli Microplate Assay*

For STEC-DT (direct stool) samples, fresh and frozen stool samples were diluted 1:3 in a diluent buffer supplied in the STEC Microplate assay. Stool samples received in a culture transport media were used directly in the STEC-DT assay without further dilution. For STEC-B (broth) samples, a tube containing 5 ml of Trypticase3 Soy Broth (BD Microbiology Systems, Sparks, MD) was inoculated with a pea size piece of stool or 50 Tl of a well-mixed stool sample in culture transport media. The tube was incubated for 24 hours at 37°C. The broth culture was diluted 1:3 in a diluent buffer supplied in the STEC Microplate assay.

*Samples for DNA Extraction*

Fresh or frozen stool samples were diluted 1:4 in STAR buffer (0.2 M citrate, 0.2 M EDTA, 0.5% ammonium lauryl sulfate, pH 5.0). Stool samples received in a culture transport media were used directly without further dilution. Chloroform was added to 10% of the total volume and the sample was vortexed. A slow speed centrifugation was performed to sediment the larger pieces. 200 Tl of the supernatant was extracted using the Total Nucleic Acid Isolation Kit and the automated MagNA Pure™ System. A positive control (in STAR buffer), and a negative control also were extracted with each batch of stool samples.

Example 2-Primers and Probes

To determine the natural sequence variation in the *stx1* or *stx2* gene, the nucleic acid sequences shown in Table 3 were aligned. An alignment of *stx* sequences is shown in Figure 1.

Table 3.

| Shiga-like toxin genes used for consensus alignment | | | |
|---|---|---|---|
| Name | Size | Organism | Accession Number |
| *stx*-dysenteriae | 2050 | *Shigella dysenteriae* | AJ271153 |
| *stx*-sonnei | 1362 | *Shigella sonnei* | AJ132761 |
| *stx1* | 1905 | Bacteriophage h30 | M23980;M21947 |
| *stx2* | 1612 | *Escherichia coli* | AF175707 |
| *stx2*-Ent | 1461 | *Enterobacter cloacae* | Z50754;U33502 |
| *stx2c* | 1499 | *Escherichia coli* | M59432 |
| *stx2d*-0111 | 1470 | *Escherichia coli* | L11078 |
| *stx2d*-Ount | 1470 | *Escherichia coli* | AF043627 |
| *stx2era* | 1263 | *Escherichia coli* | U72191 |
| *stx2f* | 1389 | *Escherichia coli* | AJ010730 |
| *stx2v&e* | 1890 | *Escherichia coli* | M21534 |

From these alignments, primers and probes directed toward *stx1* or *stx2* were designed (Table 4). Relative to the sequence shown in GenBank Accession No. M23980, the positions of the *stx1* primers were 735 to 752, and 923 to 943 on the opposite strand, while the positions of the *stx1* probes were 877 to 896 and 898 to 918. Relative to the sequence shown in GenBank Accession No. AF175707, the positions of the *stx2* primers were 365 to 380, and 549 to 569 on the opposite strand, while the positions of the *stx2* probes were 417 to 442 and 444 to 465. The *stx1* amplification product was 209 bp in length, while the *stx2* amplification product was 205 bp in length.

### Table 4. Sequences of *stx1* and *stx2* primers and probes

| | | | |
|---|---|---|---|
| Primers | *stx1* | 5'-CAA GAG CGA TGT TAC GGT-3' | 1 |
| | | 5'-AAT TCT TCC TAC ACG AAC AGA-3' | 2 |
| | *stx2* | 5'-GGG ACC ACA TCG GTG T-3' | 5 |
| | | 5'-CGG GCA CTG ATA TAT GTG TAA-3' | 6 |
| | | | SEQ ID NO: |
| Probes | *stx1* | 5'-CTG GGG AAG GTT GAG TAG CG-FITC-3' | 3 |
| | | 5'-Red640 CCT GCC TGA CTA TCA TGG ACA-PO$_4$-3' | 4 |
| | *stx2* | 5'-CTG TGG ATA TAC GAG GGC TTG ATG TC-FITC-3' | 7 |
| | | 5'-Red640 ATC AGG CGC GTT TTG ACC ATC T-PO$_4$-3' | 8 |

*stx1* or *stx2* primers were synthesized by the Mayo Core Facility on a 0.2 nm scale, and were quantitated by UV absorption at 260 nm and mixed together to make a solution containing 25 µM of each primer.

Probes were synthesized by IT Biochem, and were dissolved in TE' to a final concentration of 20 µM (supplied with the probes and resuspended according to manufacturer's instructions). The concentration of oligonucleotides and dye was double-checked by UV absorption using the following equations (*Biochemica* 1:5-8, 1999):

$$[dye] = \frac{A_{dye}}{E_{dye}} \qquad\qquad [oligo] = \frac{A_{260} - \left( A_{260} \times \dfrac{E_{260(dye)}}{E_{dye}} \right)}{\dfrac{10^6}{nmol/A_{260}}}$$

Example 3— Detection Assays

*Detection by STEC*

The ProSpecT® Shiga Toxin *E. coli* Microplate Assay is a solid phase immunoassay for the detection of Shiga-like toxins. The wells of the microplate are coated with polyclonal anti-Shiga-like toxin 1 & 2 antibody. The toxins present in a positive specimen are bound to the antibody and sandwiched with an enzyme-conjugated second antibody. The substrate for the enzyme-conjugate produces a color reaction product in a positive reaction and the color development is detected spectrophotometrically. In a negative reaction, no toxin is present to bind to the enzyme conjugate and no color product develops.

*Detection by LightCycler PCR*

For LightCycler amplification to detect Shiga-like toxin-producing *E. coli* organisms in a stool sample, the following protocol is followed. The LightCycler *stx* master mix (Table 5) is thawed, vortexed briefly, and centrifuged for 1 minute at 20,800 xg. The time reagents are left at room temperature was minimized. The LightCycler carousel was loaded with one cuvette per sample, two cuvettes for positive controls and the appropriate number of cuvettes to total 5-10% negative controls. 15 µl of the LightCycler *stx* master mix was added to each cuvette. 5 µl of the extracted sample supernatant from the MagNA Pure cartridge was added to each LightCycler cuvette.

The carousel containing the samples was centrifuged in the LightCycler Carousel Centrifuge. The carousel was placed in the LightCycler thermocycler and the LightCycler *stx* program was run. The cycling steps were complete in approximately one hour. After completion of the cycling, cuvettes were removed from the carousel with the cuvette extractor. The carousel was decontaminated in 10% bleach for 10 minutes, rinsed well with de-ionized water, and dried.

The data is analyzed using the LightCycler Software. A PCR melting analysis was used to differentiate *stx1* from *stx2* based on the Tm of the FRET probes. The probes targeting the *stx1* gene melt at 57 + 2°C, and the probes targeting the *stx2* gene melt at 66 ± 2°C.

A sample with a melting peak at the same location as the positive control was interpreted as positive. Positive samples were reported as positive for the presence of Shiga toxin- or Shiga-like toxin-producing organisms.

A sample in which the melting curve was not above baseline was negative for the presence of *E. coli stx* DNA. A negative result does not negate the presence of the organism or active disease.

Table 5.

| LightCycler *stx* Master Mix (100 reactions) | | | |
|---|---|---|---|
| Ingredient | Stock | Mix | T1 |
| Water | | | 903 |
| MgC12 | 50 mM | 4 mM | 160 |
| 10X buffer | 10X | 1X | 200 |
| Primers- *stx1* | 25 TM | 0.5 TM | 40 |
| Primers- *stx2* | 25 TM | 0.5 TM | 40 |
| Platinum Taq | 5 U/T1 | 0.03 U/T1 | 12 |
| dNTP plus | 10 mM | 0.2 mM | 40 |
| BSA | 2% | 0.025% | 25 |
| HK-UNG | 10% | 0.2% | 40 |
| Probe-*stx1*-FL | 20 TM | 0.1 TM | 10 |
| Probe-*stx1*-8640 | 20 TM | 0.1 TM | 10 |
| Probe-*stx2*-FL | 20 TM | 0.1 TM | 10 |
| Probe-*stx2*-R640 | 20 TM | 0.1 TM | 10 |
| Total volume -> | | | 1500 |

Conditions used for the real-time PCR using the LightCycler instrument to detect Shiga toxin or Shiga-like toxin in biological samples are shown in Table 6. The gains were set at 1, 5, and 15 for channels F1, F2, and F3, respectively.

Table 6.

| PCR Cycling Conditions for the Lightcycler *stx* Assay | | | | | | |
|---|---|---|---|---|---|---|
| Program Name/ Analysis mode | Analysis mode | Cycles | Temp (°C) | Time (sec) | Temp Transition Rate (°C/sec) | Signal Acquisition |
| UNG | None | 1 | 37 | 300 | 20 | None |
| | | | 95 | 180 | 20 | None |
| PCR | Quant. | 40 | 95 | 0 | 20 | None |
| | | | 55 | 10 | 20 | Single |
| | | | 72 | 14 | 20 | None |
| Melt Analysis | Melt | 1 | 95 | 0 | 20 | None |
| | | | 50 | 60 | 2 | None |
| | | | 80 | 0 | 0.2 | Continuous |
| Cool | None | 1 | 35 | 0 | 20 | None |

Example 4—Results

Of the 147 stool samples tested for the presence of the *stx* genes by ProspectT® Shiga Toxin *E. coli* Detection Assay (STEC-DT (direct), STEC-B (broth)) and the LightCycler *stx* assay, 6 (4%) were positive and 127 (86%) were negative by all assays. A total of 12 stool samples were positive with the LightCycler *stx* assay. Seven samples contained the *stx1* gene and 5 samples contained the *stx2* gene. Six of the 12 samples that were positive by the LightCycler *stx* assay were negative by both STEC-DT and STEC-B. Another one of the 12 samples that was positive by the LightCycler *stx* assay was positive by STEC-B but negative by STEC-DT. Results are summarized in Tables 7 and 8.

Seven samples were negative for *stx* genes with the LightCycler *stx* assay but were positive with STEC-DT. After a 24-hour incubation in STEC-B, this number decreased to 4 samples that were positive by STEC-DT. The addition of a 24-hour incubation of the samples in STEC-B decreased the number of false positive results obtained with STEC-DT.

Patient history was obtained on 8 of the 10 discrepant results. Four of the six specimens that were *stx* positive by LightCycler but negative by STEC-B were from patients that appeared to be infected with Shiga-like toxin-producing *E. coli* based on patient history. The four specimens that were *stx* negative by LightCycler but were *stx* positive by STEC-B were from patients that did not appear to be infected with Shiga-like toxin-producing *E. coli* based on patient history. Results of discrepant LightCycler *stx* and STEC relative to the presumptive infectious state based on the patient history demonstrated the LightCycler *stx* assay was more sensitive and specific than either antigen method (Table 9).

Table 7.

| LightCycler *stx* assay vs. STEC-DT | | | | |
|---|---|---|---|---|
| | | STEC-DT | | |
| | | Positive | Negative | Total |
| LC *stx* Assay | Positive | 6 (2) | 7 | 13 (2) |
| | Negative | 7 (3) | 127 | 134 (3) |
| | Total | 13 (5) | 134 | 147 (5) |

Table 8.

| LightCycler *stx* assay vs. STEC-B | | | | |
|---|---|---|---|---|
| | | STEC-B (24 hours) | | |
| | | Positive | Negative | Total |
| LC *stx* Assay | Positive | 7 (1) | 6 | 13 (1) |
| | Negative | 4 (4) | 130 | 134 (4) |
| | Total | 11 (5) | 136 | 147 (5) |

Table 9.

| Analysis of Samples with Discrepant LightCycler, STEC-DT and STEC-B Results | | | | |
|---|---|---|---|---|
| Patient No. | LightCycler Result | STEC-D Result | STEC-B Result | Patient History |
| 1 | *stx2* | Negative | Negative | Bloody diarrhea |
| 2 | *stx1* | Negative | Negative | Bloody diarrhea |
| 3 | *stx2* | Negative | Negative | Bloody diarrhea |
| 4 | *stx2* | Negative | Negative | Bloody diarrhea |
| 5 | Negative | Positive | Negative | Diarrhea with cancer chemotherapy |
| 6 | Negative | Positive | Negative | No diarrhea |
| 7 | Negative | Positive | Negative | No diarrhea |
| 8 | Negative | Positive | Negative | *C. jejuni* isolated |

Example 5—Quality Control

Positive controls of *E. coli stx1* (ATCC #35150) and *stx2* (patient isolate) in STAR buffer were processed by an appropriate extraction method and analyzed in each clinical run using the LightCycler *stx* assay. A melting curve analysis was used to differentiate the Shiga-like toxin nucleic acid sequences.

Positive controls were prepared as follows. Every month, the *stx1* or *stx2* strains were subcultured on blood agar plates. A McFarland 1 (3.0 x 10$^8$ organisms/ml sterile water) was prepared for each strain. The McFarland 1 was diluted 1:3 (300 Tl of McFarland 1 + 600 Tl sterile water) to make a 1.0 x 10$^8$/ml solution. A final stock solution (1000 cells/T1) for positive controls was prepared by adding 100 Tl of 1.0 x 10$^8$/ml dilution to 9900 T1 sterile water, labeled with a one-month expiration date and stored at 2-8°C. A positive control for each run was made with 20 µl of the stock solution to 180 µl STAR buffer and extrated with the MagNA Pure.

STAR buffer alone was used as the negative control. Negative controls made up 5-10% of each clinical run and were interspersed with patient samples.

The analytical detection limit of the Lightcycler *stx* assay was determined to be 10 organisms per Tl or 50 organisms per reaction. The rate of inhibition from fresh stool samples was determined to be 5%. No cross-reaction was noted using a panel of 54 different normal stool isolates, pathogenic isolates, and other *E. coli* species.

**OTHER EMBODIMENTS**

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

**Claims**

1.  A method for detecting the presence or absence of one or more Shiga toxin- or Shiga-like toxin-producing organisms in a biological sample from an individual, said method comprising:

    performing at least one cycling step, wherein a cycling step comprises an amplifying step and a hybridizing step, wherein said amplifying step comprises contacting said sample with a pair of *stx1* primers to produce an amplification product if a nucleic acid molecule encoding a Shiga toxin- or Shiga-like toxin is present in said sample, wherein said hybridizing step comprises contacting said sample with a pair of *stx1* probes, wherein the members of said pair of *stx1* probes hybridize to said amplification product within no more than five nucleotides of each other, wherein a first *stx1* probe of said pair of *stx1* probes is labeled with a donor fluorescent moiety and wherein a second *stx1* probe of said pair of *stx1* probes is labeled with a corresponding acceptor fluorescent moiety; and
    detecting the presence or absence of fluorescence resonance energy transfer (FRET) between said donor fluorescent moiety of said first *stx1* probe and said acceptor fluorescent moiety of said second *stx1* probe,

    wherein the presence of FRET is indicative of the presence of one or more Shiga toxin- or Shiga-like toxin-producing organisms in said biological sample, and wherein the absence of FRET is indicative of the absence of a Shiga toxin- or Shiga-like toxin-producing organism in said biological sample.

**2.** The method of claim 1, wherein said organism is *E. coli* and said toxin is a Shiga-like toxin.

**3.** The method of claim 1, wherein said pair of *stx1* primers comprises a first *stx1* primer and a second *stx1* primer, wherein said first *stx1* primer comprises the sequence 5'-CAA GAG CGA TGT TAC GGT-3' (SEQ ID NO:1), and wherein said second *stx1* primer comprises the sequence

<div align="center">5'-AAT TCT TCC TAC ACG AAC AGA-3' (SEQ ID NO:2).</div>

**4.** The method of claim 1, wherein said first *stx1* probe comprises the sequence 5'-CTG GGG AAG GTT GAG TAG CG-3' (SEQ ID NO:3), and wherein said second *stx1* probe comprises the sequence

<div align="center">5'-CCT GCC TGA CTA TCA TGG ACA-3' (SEQ ID NO:4).</div>

**5.** The method of claim 1, wherein the members of said pair of *stx1* probes hybridize within no more than two nucleotides of each other.

**6.** The method of claim 1, wherein the members of said pair of *stx1* probes hybridize within no more than one nucleotide of each other.

**7.** The method of claim 1, wherein said donor fluorescent moiety is fluorescein.

**8.** The method of claim 1, wherein said acceptor fluorescent moiety is selected from the group consisting of LC-Red 640, LC-Red 705, Cy5, and Cy5.5.

**9.** The method of claim 1, wherein said detecting step comprises exciting said biological sample at a wavelength absorbed by said donor fluorescent moiety and visualizing and/or measuring the wavelength emitted by said acceptor fluorescent moiety.

**10.** The method of claim 1, wherein said detecting comprises quantitating said FRET.

**11.** The method of claim 1, wherein said detecting step is performed after each cycling step.

**12.** The method of claim 1, wherein said detecting step is performed in real-time.

**13.** The method of claim 1, further comprising determining the melting temperature between one or both of said *stx1* probe(s) and said amplification product, wherein said melting temperature confirms said presence or said absence of said Shiga toxin- or Shiga-like toxin-producing organism.

**14.** The method of claim 1, wherein the presence of said FRET within 50 cycles is indicative of the presence of a Shiga toxin- or Shiga-like toxin-producing organism in said individual.

**15.** The method of claim 1, wherein the presence of said FRET within 40 cycles is indicative of the presence of a Shiga toxin- or Shiga-like toxin-producing organism in said individual.

**16.** The method of claim 1, wherein the presence of said FRET within 30 cycles is indicative of the presence of a Shiga toxin- or Shiga-like toxin-producing organism in said individual.

**17.** The method of claim 1, further comprising: preventing amplification of a contaminant nucleic acid.

**18.** The method of claim 17, wherein said preventing comprises performing said amplification step in the presence of uracil.

**19.** The method of claim 18, wherein said preventing further comprises treating said biological sample with uracil-DNA glycosylase prior to a first amplifying step.

**20.** The method of claim 1, wherein said biological sample is selected from the group consisting of stool samples and body fluids.

**21.** The method of claim 1, wherein said cycling step is performed on a control sample.

**22.** The method of claim 21, wherein said control sample comprises said nucleic acid molecule encoding a Shiga toxin or Shiga-like toxin.

**23.** The method of claim 1, wherein said cycling step uses a pair of control primers and a pair of control probes, wherein said control primers and said control probes are other than said *stx1* primers and said *stx1* probes, respectively, wherein a control amplification product is produced if control template is present in said sample, wherein said control probes hybridize to said control amplification product.

**24.** An article of manufacture, comprising:

a pair of *stx1* primers;
a pair of *stx1* probes; and
a donor fluorescent moiety and a corresponding fluorescent moiety.

**25.** The article of manufacture of claim 24, wherein said pair of *stx1* primers comprises a first *stx1* primer and a second *stx1* primer, wherein said first *stx1* primer comprises the sequence
5'-CAA GAG CGA TGT TAC GGT-3' (SEQ ID NO:1), and wherein said second *stx1* primer comprises the sequence

5'-AAT TCT TCC TAC ACG AAC AGA-3' (SEQ ID NO:2).

**26.** The article of manufacture of claim 24, wherein said pair of *stx1* probes comprises a first *stx1* probe and a second *stx1* probe, wherein said first *stx1* probe comprises the sequence
5'-CTG GGG AAG GTT GAG TAG CG-3' (SEQ ID NO:3), and wherein said second *stx1* probe comprises the sequence

5'-CCT GCC TGA CTA TCA TGG ACA-3' (SEQ ID NO:4).

**27.** The article of manufacture of claim 24, wherein said pair of *stx1* probes comprises a first *stx1* probe labeled with said donor fluorescent moiety and a second *stx1* probe labeled with said corresponding acceptor fluorescent moiety.

**28.** The article of manufacture of claim 24, further comprising a package label or package insert having instructions thereon for using said pair of *stx1* primers and said pair of *stx1* probes to detect the presence or absence of one or more Shiga toxin- or Shiga-like toxin-producing organisms in a biological sample.

**29.** A method for detecting the presence or absence of one or more Shiga-like toxin-producing *E. coli* organisms in a biological sample from an individual, said method comprising:

performing at least one cycling step, wherein a cycling step comprises an amplifying step and a hybridizing step, wherein said amplifying step comprises contacting said sample with a pair of *stx2* primers to produce a *stx2* amplification product if an *E. coli* Shiga-like toxin *stx2* nucleic acid molecule is present in said sample, wherein said hybridizing step comprises contacting said sample with a pair of *stx2* probes, wherein the members of said pair of *stx2* probes hybridize to said amplification product within no more than five nucleotides of each other, wherein a first *stx2* probe of said pair of *stx2* probes is labeled with a donor fluorescent moiety and wherein a second *stx2* probe of said pair of *stx2* probes is labeled with a corresponding acceptor fluorescent moiety; and

detecting the presence or absence of fluorescence resonance energy transfer (FRET) between said donor fluorescent moiety of said first *stx2* probe and said acceptor fluorescent moiety of said second *stx2* probe,

wherein the presence of FRET is indicative of the presence of one or more Shiga-like toxin-producing *E. coli* organisms in said biological sample, and wherein the absence of FRET is indicative of the absence of a Shiga-like toxin-producing *E. coli* organism in said biological sample.

**30.** The method of claim 29, wherein said pair of *stx2* primers comprises a first *stx2* primer and a second *stx2* primer, wherein said first *stx2* primer comprises the sequence

5'-GGG ACC ACA TCG GTG T-3' (SEQ ID NO:5), and wherein said second *stx2* primer comprises the sequence

5'-CGG GCA CTG ATA TAT GTG TAA-3' (SEQ ID NO:6).

**31.** The method of claim 29, wherein said first *stx2* probe comprises the sequence

5'-CTG TGG ATA TAC GAG GGC TTG ATG TC-3' (SEQ ID NO:7), and wherein said second *stx2* probe comprises the sequence

5'-ATC AGG CGC GTT TTG ACC ATC T-3' (SEQ ID NO:8).

**32.** The method of claim 1, further comprising:

performing at least one cycling step, wherein a cycling step comprises an amplifying step and a hybridizing step, wherein said amplifying step comprises contacting said sample with a pair of *stx2* primers to produce a *stx2* amplification product if an *E. coli* Shiga-like toxin *stx2* nucleic acid molecule is present in said sample, wherein said hybridizing step comprises contacting said sample with a pair of *stx2* probes, wherein the members of said pair of *stx2* probes hybridize to said amplification product within no more than five nucleotides of each other, wherein a first *stx2* probe of said pair of *stx2* probes is labeled with a donor fluorescent moiety and wherein a second *stx2* probe of said pair of *stx2* probes is labeled with a corresponding acceptor fluorescent moiety; and

detecting the presence or absence of fluorescence resonance energy transfer (FRET) between said donor fluorescent moiety of said first *stx2* probe and said acceptor fluorescent moiety of said second *stx2* probe,

wherein the presence of FRET is indicative of the presence of one or more Shiga-like toxin-producing *E. coli* organisms in said biological sample, and wherein the absence of FRET is indicative of the absence of a Shiga-like toxin-producing *E. coli* organisms in said biological sample.

**33.** An article of manufacture, comprising:

a pair of *stx2* primers;
a pair of *stx2* probes; and
a donor fluorescent moiety and a corresponding fluorescent moiety.

**34.** The article of manufacture of claim 33, wherein said pair of *stx2* primers comprises a first *stx2* primer and a second *stx2* primer, wherein said first *stx2* primer comprises the sequence

5'-GGG ACC ACA TCG GTG T-3' (SEQ ID NO:5), and wherein said second *stx2* primer comprises the sequence

5'-CGG GCA CTG ATA TAT GTG TAA-3' (SEQ ID NO:6).

**35.** The article of manufacture of claim 33, wherein said pair of *stx2* probes comprises a first *stx2* probe and a second *stx2* probe, wherein said first *stx2* probe comprises the sequence
5'-CTG TGG ATA TAC GAG GGC TTG ATG TC-3' (SEQ ID NO:7), and wherein said second *stx2* probe comprises the sequence

<div align="center">

### 5'-ATC AGG CGC GTT TTG ACC ATC T-3' (SEQ ID NO:8).

</div>

36. The article of manufacture of claim 33, wherein said pair of *stx2* probes comprises a first *stx2* probe labeled with said donor fluorescent moiety and a second *stx2* probe labeled with said corresponding acceptor fluorescent moiety.

37. The article of manufacture of claim 33, further comprising a package label or package insert having instructions thereon for using said pair of *stx2* primers and said pair of *stx2* probes to detect the presence or absence of Shiga-like toxin-producing *E. coli* organism in a biological sample.

38. A method for detecting the presence or absence of one or more Shiga toxin- or Shiga-like toxin-producing organisms in a biological sample from an individual, said method comprising:

    performing at least one cycling step, wherein a cycling step comprises an amplifying step and a hybridizing step, wherein said amplifying step comprises contacting said sample with a pair of *stx1* primers to produce an amplification product if a nucleic acid molecule encoding Shiga toxin or Shiga-like toxin is present in said sample, wherein said hybridizing step comprises contacting said sample with a *stx1* probe, wherein said *stx1* probe is labeled with a donor fluorescent moiety and a corresponding acceptor fluorescent moiety; and detecting the presence or absence of fluorescence resonance energy transfer (FRET) between said donor fluorescent moiety and said acceptor fluorescent moiety of said *stx1* probe,

    wherein the presence or absence of FRET is indicative of the presence or absence of one or more Shiga toxin- or Shiga-like toxin-producing organisms in said sample.

39. The method of claim 38, wherein said amplification employs a polymerase enzyme having 5' to 3' exonuclease activity.

40. The method of claim 39, wherein said donor and acceptor fluorescent moieties are within no more than 5 nucleotides of each other on said probe.

41. The method of claim 40, wherein said acceptor fluorescent moiety is a quencher.

42. The method of claim 38, wherein said *stxl* probe comprises a nucleic acid sequence that permits secondary structure formation, wherein said secondary structure formation results in spatial proximity between said donor and said acceptor fluorescent moiety.

43. The method of claim 42, wherein said acceptor fluorescent moiety is a quencher.

44. A method for detecting the presence or absence of one or more Shiga toxin- or Shiga-like toxin-producing organisms in a biological sample from an individual, said method comprising:

    performing at least one cycling step, wherein a cycling step comprises an amplifying step and a dye-binding step, wherein said amplifying step comprises contacting said sample with a pair of *stx1* primers to produce an amplification product if a nucleic acid molecule encoding Shiga toxin or Shiga-like toxin is present in said sample, wherein said dye-binding step comprises contacting said amplification product with a double-stranded nucleic acid binding dye; and detecting the presence or absence of binding of said double-stranded nucleic acid binding dye to said amplification product,

    wherein the presence of binding is indicative of the presence of one or more Shiga toxin- or Shiga-like toxin-producing organisms in said sample, and wherein the absence of binding is indicative of the absence of a Shiga toxin- or Shiga-like toxin-producing organism in said sample.

45. The method of claim 44, wherein said double-stranded nucleic acid binding dye is selected from the group consisting of SYBRGreenI®, SYBRGold®, and ethidium bromide.

46. The method of claim 45, further comprising determining the melting temperature between said amplification product and said double-stranded nucleic acid binding dye, wherein said melting temperature confirms said presence or absence of said Shiga toxin- or Shiga-like toxin-producing organism.

```
                    ....|....| ....|....| ....|....| ....|....| ....|....|
                          10         20         30         40         50
Stx1                ATGAAAATAA TTATTTTTAG AGTGCTAACT TTTTTCTTTG TTATCTTTTC
Stx-dysenteria      ATGAAAATAA TTATTTTTAG AGTGCTAACT TTTTTCTTTG TTATCTTTTC
Stx-sonnei          ATGAAAATAA TTATTTTTAG AGTGCTAACT TTTTTCTTTG TTATCTTTTC
Stx2                ATGAAG-TGT ATATTATTTA AATGGGTACT GTGCCTGTTA CTGGGTTTTT
Stx2f               ATGCGA-CAT ATATTATTAA AGCTGGTGTT GTTTTTTTGT GTTTGCTTGT
Stx2c               ATGAAG-TGT ATATTATTTA AATGGGTACT GTGCCTGTTA CTGGGCTTTT
Stx2d-0111          ATGAAG-TGT ATATTATTTA AATGGGTACT GTGCCTGTTA CTGGGTTTTT
Stx2d-Ount          ATGAAG-TGT ATATTGTTAA AATGGGTACT GTGCCTGTTA CTGGGCTTTT
Stx2v&e             ATGAAG-TGT ATATTGTTAA AGTGGATACT GTGTCTGTTA CTGGGTTTTT
Stx2era             ATGGAG-TGT ATATTGTTAA AGTGGATACT GTGTCTGTTA CTGGGTTTTT
Stx2-Ent            ATGAAG-TGT ATATTATTTA AATGGGTACT GTGCCTGTTA CTGGGTTTTT


                    ....|....| ....|....| ....|....| ....|....| ....|....|
                          60         70         80         90        100
Stx1                AGTTAATGTG GTG-GCGAAG GAATTTACCT TAGACTTCTC GACTGCAAAG
Stx-dysenteria      AGTTAATGTG GTG-GCGAAG GAATTTACCT TAGACTTCTC GACTGCAAAG
Stx-sonnei          AGTTAATGTG GTT-GCGAAG GAATTTACCT TAGACTTCTC GACTGCAAAG
Stx2                CTTCGGTATC CTATTCCCGG GAGTTACGA TAGACTTTTC GACCCAACAA
Stx2f               CTTCAGCATC TTATGCAGAT GAGTTACTG TGGATTTCTC TTCGCAAAAG
Stx2c               CTTCGGTATC CTATTCCCGG GAATTTACGA TAGACTTTTC GACTCAACAA
Stx2d-0111          CTTCGGTATC CTATTCCCGG GAGTTTATGA TAGACTTTTC GACCCAACAA
Stx2d-Ount          CTTCGGTATC CTATTCCCGG GAATTTACGA TAGACTTTTC GACTCAACAA
Stx2v&e             CTTCGGTATC CTATTCCCAG GAGTTTACGA TAGACTTTTC GACTCAACAA
Stx2era             CTTCGGTATC CTATTCCCAG GAGTTTACGA TAGACTTTCC GACTCAACAA
Stx2-Ent            CTTCGGTATC CTATTCCCGG GAATTTACGA TAGACCTTTC GACCCAACAA


                    ....|....| ....|....| ....|....| ....|....| ....|....|
                         110        120        130        140        150
Stx1                ACGTATGTAG ATTCGCTGAA TGTCATTCGC TCTGCAATAG GTACTCCATT
Stx-dysenteria      ACGTATGTAG ATTCGCTGAA TGTCATTCGC TCTGCAATAG GTACTCCATT
Stx-sonnei          ACGTATGTAG ATTCGCTGAA TGTCATTCGC TCTGCAATAG GTACTCCATT
Stx2                AGTTATGTCT CTTCGTTAAA TAGTATACGG ACAGAGATAT CGACCCCTCT
Stx2f               AGCTATGTTG ATTCATTGAA TAGTATAAGG TCGGCAATAT CCACTCCACT
Stx2c               AGTTATGTAT CTTCGTTAAA TAGTATACGG ACAGAGATAT CGACCCCTCT
Stx2d-0111          AGTTATGTCT CTTCGTTAAA TAGTATACGG ACAGAGATAT CGACCCCTCT
Stx2d-Ount          AGTTATGTCT CTTCGTTAAA TTGTATACGG ACAGAAATAT CGACCCCACT
Stx2v&e             AGTTATGTAT CTTCGTTAAA TAGTATACGG ACAGCGATAT CGACCCCTCT
Stx2era             AGTTATGTAT CTTCGTTAAA TAGTATACGG ACAGCGATAT CGACCCCTCT
Stx2-Ent            AGTTATGTCT CTTCGTTAAA TAGTATACGG ACAGAGATAT CGACCCCTCT


                    ....|....| ....|....| ....|....| ....|....| ....|....|
                         160        170        180        190        200
Stx1                ACAGACTATT TCATCAGGAG GTACGTCTTT ACTGATGATT GATAGTGGCT
Stx-dysenteria      ACAGACTATT TCATCAGGAG GTACGTCTTT ACTGATGATT GATAGTGGCW
Stx-sonnei          ACAGACTATT TCATCAGGAG GTACGTCTTT ACTGATGATT GATAGTGGCA
                                         Stx2-365 ->
Stx2                TGAACATATA TCTCAGGGGA CCACATCGGT GTCTGTTATT AACCACACCC
Stx2f               TGGAAATATA TCTCAGGGTG GTGTTTCTGT TTCAGTAATT AATCATGTTC
Stx2c               TGAACATATA TCTCAGGGGA CCACATCGGT GTCTGTTATT AACCACACCC
Stx2d-0111          TGAACATATA TCTCAGGGGA CCACATCGGT GTCTGTTATT AACCACACCC
Stx2d-Ount          TGAACATATA TCTCAGGGGA CCACATCGGT ATCTGTTATT AACCACACCC
Stx2v&e             TGAACATATA TCTCAGGGAG CTACATCGGT ATCCGTTATT AATCATACAC
Stx2era             TGAACATATA TCTCAGGGAG CTACATCGGT ATCCGTTATT AATCATACAC
Stx2-Ent            TGAACATATA TCTCAGGGGA CCACATCGGT GTCTGTTATT AACCACACCC
```

Figure 1-1

```
              ....|....| ....|....| ....|....| ....|....| ....|....|
                 210        220        230        240        250
Stx1          CAGGGGATAA TTTGTTTGCA GTTGATGTCA GAGGGATAGA TCCAGAGGAA
Stx-dysenteria CAGGGGATAA TTTGTTTGCA GTTGATGTCA GAGGGATAGA TCCAGAGGAA
Stx-sonnei    CAGGGGATAA TTTGTTTGCA GTTGATGTCA GAGGGATAGA TCCAGAGGAA
                                        Stx2-F                    stx2-R
Stx2          CACCGGGCAG TTATTTTGCT GTGGATATAC GAGGGCTTGA TGTCTATCAG
Stx2f         CAGGCGGAAA CTATATATCA TTGAATGTTA GAGGCCTTGA TCCATATAGC
Stx2c         CACCGGGCAG TTATTTTGCT GTGGATATAC GAGGGCTTGA TGTCTATCAG
Stx2d-0111    CACCGGGCAG TTATTTTGCT GTGGATATAC GAGGGCTTGA TGTCTATCAG
Stx2d-Ount    CACCGGGCAG TTATTTTGCT GTGGATATAC GAGGGCTTGA TGTCTATCAG
Stx2v&e       CACCAGGAAG TTATATTTCC GTAGGTATAC GAGGGCTTGA TGTTTATCAG
Stx2era       CACCAGGAAG TTATATTTCC GTAGGTATAC GAGGGCTTGA TGTTTATCAG
Stx2-Ent      CGCCGGGCAG TTATTTTGCT GTGGATATAC GAGGGCTTGA TGTCTATCAG


              ....|....| ....|....| ....|....| ....|....| ....|....|
                 260        270        280        290        300
Stx1          GGGCGGTTTA ATAATCTACG GCTTATTGTT GAACGAAATA ATTTATATGT
Stx-dysenteria GGGCGGTTTA ATAATCTACG GCTTATTGTT GAACGAAATA ATTTATATGT
Stx-sonnei    GGGCGGTTTA ATAATCTACG GCTTATTGTT GAACGAAATA ATTTATATGT
Stx2          GCGCGTTTTG ACCATCTTCG TCTGATTATT GAGCAAAATA ATTTATATGT
Stx2f         GAGAGATTTA ACCACCTCCG TTTAATAATG GAACGGAATA ACTTATATGT
Stx2c         GCGCGTTTTG ACCATCTTCG TCTGATTATT GAGCAAAATA ATTTATATGT
Stx2d-0111    GCGCGTTTTG ACCATCTTCG TCTGATTATT GAGCAAAATA ATTTATATGT
Stx2d-Ount    GCGCGTTTTG ACCATCTTCG TCTGATTATT GAGCAAAATA ATTTATATGT
Stx2v&e       GAGCGTTTTG ACCATCTTCG TCTGATTATT GAACGAAATA ATTTATATGT
Stx2era       GAGCGTTTTG ACCATCTTCG TCCGATTATT GAACGAAATA ATTTATATGT
Stx2-Ent      GCGCGTTTTG ACCATCTTCG TCTGATTATT GAGCAAAATA ATTTATATGT


              ....|....| ....|....| ....|....| ....|....| ....|....|
                 310        320        330        340        350
Stx1          GACAGGATTT GTTAACAGGA CAAATAATGT TTTTTATCGC TTTGCTGATT
Stx-dysenteria GACAGGATTT GTTAACAGGA CAAATAATGT TTTTTATCGC TTTGCTGATT
Stx-sonnei    GACAGGATTT GTTAACAGGA CAAATAATGT TTTTTATCGC TTTGCTGATT
Stx2          GGCCGGGTTC GTTAATACGG CAACAAATAC TTTCTACCGT TTTTCAGATT
Stx2f         TGCAGGCTTT ATTAATACTG AAACGAATAC CTTTTACAGA TTCTCCGATT
Stx2c         GGCCGGGTTC GTTAATACGG CAACAAATAC TTTCTACCGT TTTTCAGATT
Stx2d-0111    GGCTGGGTTC GTTAATACGG CAACAAATAC TTTCTACCGT TTTTCAGATT
Stx2d-Ount    GGCCGGATTC GTTAATACGG CAACAAATAC TTTCTACAGA TTTTCAGATT
Stx2v&e       GGCTGGATTT GTTAATACGA CAACAAATAC TTTCTACAGA TTTTCAGATT
Stx2era       GGCTGGATTT GTTAATACGA CAACAAATAC TTTCTACAGA TTTTCAGATT
Stx2-Ent      GGCCGGGTTC GTTAATACGG CAACAAATAC TTTCTACCGT TTTTCAGATT


              ....|....| ....|....| ....|....| ....|....| ....|....|
                 360        370        380        390        400
Stx1          TTTCACATGT TACCTTTCCA GGTACAACAG CGGTTACATT GTCTGGTGAC
Stx-dysenteria TTTCACATGT TACCTTTCCA GGTACAACAG CGGTTACATT GTCTGGTGAC
Stx-sonnei    TTTCACATGT TACCTTTCCA GGTACAACAG CGGTTACATT GTCTGGTGAC
                 <- stx2-549
Stx2          TTACACATAT ATCAGTGCCC GGTGTGACAA CGGTTTCCAT GACAACGGAC
Stx2f         TCTCACATAT TTCAGTGCCT GATGTGATAA CTGTTTCCAT GACGACGGAC
Stx2c         TTACACATAT ATCAGTGCCC GGTGTGACAA CGGTTTCCAT GACAACGGAC
Stx2d-0111    TTACACATAT ATCAGTGCCC GGTGTGACAA CGGTTTCCAT GACAACGGAC
Stx2d-Ount    TTGCACATAT ATCAGTGCCC GGTGTGACAA CTGTTTCCAT GACAACGGAC
Stx2v&e       TTGCACATAT ATCATTGCCC GGTGTGACAA CTATTTCCAT GACAACGGAC
Stx2era       TTGCACATAT ATCATTGCCT GGTGTGACAA CTATTTCCAT GACAACGGAC
Stx2-Ent      TTACACATAT ATCAGTGCCC GGTGTGACAA CGGTTTCCAT GACAACGGAC
```

Figure 1-2

26

```
                ....|....| ....|....| ....|....| ....|....| ....|....|
                    410        420        430        440        450
Stx1            AGTAGCTATA CCACGTTACA GCGTGTTGCA GGGATCAGTC GTACGGGGAT
Stx-dysenteria  AGTAGCTATA CCACGTTACA GCGTGTTGCA GGGATCAGTC GTACGGGGAT
Stx-sonnei      AGTAGCTATA CCACGTTACA GCGTGTTGCA GGGATCAGTC GTACGGGGAT
Stx2            AGCAGTTATA CCACTCTGCA ACGTGTCGCA GCGCTGGAAC GTTCCGGAAT
Stx2f           AGCAGTTATT CATCATTACA GCGAATCGCA GATCTGGAAC GTACAGGGAT
Stx2c           AGCAGTTATA CCACTCTGCA ACGTGTCGCA GCGCTGGAAC GTTCCGGAAT
Stx2d-0111      AGCAGTTATA CCACTCTGCA ACGTGTCGCA GCGCTGGAAC GTTCCGGAAT
Stx2d-Ount      AGCAGTTATA CCACTCTGCA ACGTGTCGCA GCGCTGGAAC GTTCCGGAAT
Stx2v&e         AGCAGTTATA CCACTCTGCA ACGTGTCGCA GCGCTGGAAC GTTCCGGAAT
Stx2era         AGCAGTTATA CCACTCTGCA ACGTGTCGCA GCGCTGGAAC GTTCCGGAAT
Stx2-Ent        AGCAGTTATA CCACTCTGCA ACGTGTCGCA GCGCTGGAAC GTTCCGGAAT


                ....|....| ....|....| ....|....| ....|....| ....|....|
                    460        470        480        490        500
Stx1            GCAGATAAAT CGCCATTCGT TGACTACTTC TTATCTGGAT TTAATGTCGC
Stx-dysenteria  GCAGATAAAT CGCCATTCGT TGACTACTTC TTATCTGGAT TTAATGTCGC
Stx-sonnei      GCAGATAAAT CGCCATTCGT TGACTACTTC TTATCTGGAT TTAATGTCGC
Stx2            GCAAATCAGT CGTCACTCAC TGGTTTCATC ATATCTGGCG TTAATGGAGT
Stx2f           GCAGATTGGG CGTCATTCAC TGGTTGGTTC ATATCTGGAT TTAATGGAGT
Stx2c           GCAAATCAGT CGTCACTCAC TGGTTTCATC ATATCTGGCG TTAATGGAGT
Stx2d-0111      GCAAATCAGT CGTCACTCAC TGGTTTCATC ATATCTGGCG TTAATGGAGT
Stx2d-Ount      GCAAATCAGT CGTCACTCAC TGGTTTCATC ATATCTGGCG TTAATGGAGT
Stx2v&e         GCAAATCAGT CGTCACTCAC TGGTTTCATC ATATCTGGCG TTAATGGAGT
Stx2era         GCAAATCAGT CGTCACTCAC TGGTTTCATC GTATCTGGCG TTAATGGAGT
Stx2-Ent        GCAAATCAGT CGTCACTCAC TGGTTTCATC ATATCTGGCG TTAATGGAGT


                ....|....| ....|....| ....|....| ....|....| ....|....|
                    510        520        530        540        550
                                                    stx1-735 ->
Stx1            ATAGTGGAAC CTCACTGACG CAGTCTGTGG CAAGAGCGAT GTTACGGTTT
Stx-dysenteria  ATAGTGGAAC CTCACTGACG CAGTCTGTGG CAAGAGCGAT GTTACGGTTT
Stx-sonnei      ATAGTGGAAC CTCACTGACG CAGTCTGTGG CAAGAGCGAT GTTACGGTTT
Stx2            TCAGTGGTAA TACAATGACC AGAGATGCAT CCAGAGCAGT TCTGCGTTTT
Stx2f           TCAGAGGACG TTCCATGACC CGCGCATCAT CCAGAGCTAT GCTGCGTTTT
Stx2c           TCAGTGGTAA TACAATGACC AGAGATGCAT CCAGAGCAGT TCTGCGTTTT
Stx2d-0111      TCAGTGGTAA TACAATGACC AGAGATGCAT CCAGAGCAGT TCTGCGTTTT
Stx2d-Ount      TTAGTGGAAA TGCCATGACC AGAGATGCAT CCAGAGCAGT TCTGCGTTTT
Stx2v&e         TCAGTGGTAA TACAATGACC AGAGATGCAT CAAGAGCAGT TCTGCGTTTT
Stx2era         TCAGTGGTAA TACAATGACC AGAGATGCAT CAAGAGCAGT TCTGCGTTTT
Stx2-Ent        TCAGTGGTAA TACAATGACC AGAGATGCAT CCAGAGCAGT TCTGCGTTTT


                ....|....| ....|....| ....|....| ....|....| ....|....|
                    560        570        580        590        600
Stx1            GTTACTGTGA CAGCTGAAGC TTTACGTTTT CGGCAAATAC AGAGGGGATT
Stx-dysenteria  GTTACTGTGA CAGCTGAAGC TTTACGTTTT CGGCAAATAC AGAGGGGATT
Stx-sonnei      GTTACTGTGA CAGCTGAAGC TTTACGTTTT CGGCAAATAC AGAGGGGATT
Stx2            GTCACTGTCA CAGCAGAAGC CTTACGCTTC AGGCAGATAC AGAGAGAATT
Stx2f           GTCACAGTGA TAGCAGAAGC TCTGCGATTC AGACAAATAC AGCGGGGATT
Stx2c           GTCACTGTCA CAGCAGAAGC CTTACGCTTC AGGCAGATAC AGAGAGAATT
Stx2d-0111      GTCACTGTCA CAGCAGAAGC CTTACGCTTC AGGCAGATAC AGGGAGAATT
Stx2d-Ount      GTCACTGTCA CAGCAGAAGC CTTACGGTTC AGGCAAATAC AGAGAGAATT
Stx2v&e         GTCACTGTCA CAGCAGAAGC CTTACGGTTC AGGCAAATAC AGAGAGAATT
Stx2era         GTCACTGTCA CAGCAGAAGC CTTACGGTTC AGGCAAATAC AGAGAGAATT
Stx2-Ent        GTCACTGTCA CAGCAGAAGC CTTACGCTTC AGGCAGATAC AGAGAGAATT
```

Figure 1-3

```
                    ....|....| ....|....| ....|....| ....|....| ....|....|
                      610        620        630        640        650
Stx1                TCGTACAACA CTGGATGATC TCAGTGGGCG TTCTTATGTA ATGACTGCTG
Stx-dysenteria      TCGTACAACA CTGGATGATC TCAGTGGGCG TTCTTATGTA ATGACTGCTG
Stx-sonnei          TCGTACAACA CTGGATGATC TCAGTGGGCG TTCTTATGTA ATGACTGCTG
Stx2                TCGTCAGGCA CTGTCTGAA- --ACTGCTCC TGTGTATACG ATGACGCCGG
Stx2f               CCGACCGGCG CTGTCTGAG- --GCATCTCC GCTTTATACA ATGACGGCTC
Stx2c               TCGTCAGGCA CTGTCTGAA- --ACTGCTCC TGTGTATACG ATGACGCCGG
Stx2d-0111          TCGTCAGGCA CTGTCTGAA- --ACTGCTCC TGTGTATACG ATGACACCGG
Stx2d-Ount          TCGTCTGGCA CTGTCTGAA- --ACTGCTCC TGTTTATACG ATGACACCGG
Stx2v&e             TCGTCTGGCA CTGTCTGAA- --ACTGCTCC TGTTTATACG ATGACGCCGG
Stx2era             TCGTCTGGCA CTGTCTGAA- --ACTGCTCC TGTTTATACG ATGACGCCGG
Stx2-Ent            TCGTCAGGCA CTGTCTGAA- --ACTGCTCC TGTGTATACG ATGACGGCGG


                    ....|....| ....|....| ....|....| ....|....| ....|....|
                      660        670        680        690        700
                                                    Stx1-F              Stx1-R
Stx1                AAGATGTTGA TCTTACATTG AACTGGGGAA GGTTGAGTAG CGTCCTGCCT
Stx-dysenteria      AAGATGTTGA TCTTACATTG AACTGGGGAA GGTTGAGTAG YGTCCTGCCT
Stx-sonnei          AAGATGTTGA TCTTACATTG AACTGGGGAA GGTTGAGTAG TGTCCTGCCT
Stx2                GAGACGTGGA CCTCACTCTG AACTGGGGGC GAATCAGCAA TGTGCTTCCG
Stx2f               AGGATGTTGA CCTTACCCTG AACTGGGGAA GAATAAGTAA TGTTCTTCCA
Stx2c               GAGACGTGGA CCTCACTCTG AACTGGGGGC GAATCAGCAA TGTGCTTCCG
Stx2d-0111          AAGAAGTGGA CCTCACACTG AACTGGGGGA GAATCAGCAA TGTGCTTCCG
Stx2d-Ount          AAGAAGTGGA CCTCACACTG AACTGGGGGA GAATCAGCAA TGTGCTTCCG
Stx2v&e             AAGACGTGGA CCTCACTCTG AACTGGGGGA GAATCAGCAA TGTGCTTCCG
Stx2era             AAGACGTGGA CCTCACTCTG AACTGGGGGA GAATCAGCAA TGTGCTTCCG
Stx2-Ent            GAGACGTGGA CCTCACTCTG AACTGGGGGC GAATCAGCAA TGTGCTTCCG


                    ....|....| ....|....| ....|....| ....|....| ....|....|
                      710        720        730        740        750
                                            <- Stx1-923
Stx1                GACTATCATG GACAAGACTC TGTTCGTGTA GGAAGAATTT CTTTTGGAAG
Stx-dysenteria      GACTATCATG GACAAGACTC TGTTCGTGTA GGAAGAATTT CTTTTGGAAG
Stx-sonnei          GACTATCATG GACAAGACTC TGTTCGTGTA GGAAGAATTT CTTTTGGAAG
Stx2                GAGTATCGGG GAGAGGATGG TGTCAGAGTG GGGAGAATAT CCTTTAATAA
Stx2f               GAGTACAGAG GAGAGGAAGG GGTAAGAATC GGTAGGATAT CTTTTAATAG
Stx2c               GAGTATCGGG GAGAGGATGG TGTCAGAGTG GGGAGAATAT CCTTTAATAA
Stx2d-0111          GAGTTTCGGG GAGAGGGGGG TGTCAGAGTG GGGCGAATAT CCTTTAATAA
Stx2d-Ount          GAGTTTCGGG GAGAGGGTGG TGTCAGAGTG GGGCGAATAT CCTTTAATAA
Stx2v&e             GAGTATCGGG GAGAGGCTGG TGTCAGAGTG GGGAGAATAT CCTTTAATAA
Stx2era             GAGTATCGGG GAGAGGCTGG TGTCAGAGTG GGGAGAATAT CCTTTAATAA
Stx2-Ent            GAGTATCGGG GAGAGGATGG TGTCAGAGTG GGGAGAATAT CCTGTAATAA


                    ....|....| ....|....| ....|....| ....|....| ....|....|
                      760        770        780        790        800
Stx1                CATTAATGCA ATTCTGGGAA GCGTGGCATT AATACTGAAT TGTCA-TCAT
Stx-dysenteria      CATTAATGCA ATTCTGGGAA GCGTGGCATT AATACTGAAT TGTCA-TCAT
Stx-sonnei          CATTAATGCA ATTCTGGGAA GCGTGGCATT AATACTGAAT TGTCA-TCAT
Stx2                TATATCAGCG ATACTGGGGA CTGTGGCCGT TATACTGAAT TGCCA-TCAT
Stx2f               TCTTTCTGCG ATTCTCGGAA GTGTTGCGGT CATCCTTAAT TGCCACTCAA
Stx2c               TATATCAGCG ATACTGGGGA CTGTGGCCGT TATACTGAAT TGCCA-TCAT
Stx2d-0111          TATATCAGCG ATACTGGGCA CAGTGGCGGT TATACTGAAT TGCCA-TCAT
Stx2d-Ount          TATATCAGCG ATACTGGGCA CAGTGGCGGT TATACTGAAT TGCCA-TCAT
Stx2v&e             TATATCAGCG ATACTTGGTA CTGTGGCCGT TATACTGAAT TGCCA-TCAT
Stx2era             TATATCAGCG ATACTTGGTA CTGTGGCCGT TATACTGAAT TGCCA-TCAT
Stx2-Ent            TATATCAGCG ATACTGGGTA CTGTGGCCGT TATACTGAAT TGCCA-TCAT
```

Figure 1-4

```
                  ....|....| ....|....| ....|....| ....|....| ....|....|
                       810        820        830        840        850
Stx1             CATGCATCGC GAGTTGCCAG AATGGCATCT GATGAGTTTC CTTCTATGTG
Stx-dysenteria   CATGCATCGC GAGTTGCCAG AATGGCATCT GATGAGTTTC CTTCTATGTG
Stx-sonnei       CATGCATCGC GAGTTGCCAG AATGGCATCT GATGAGTTTC CTTCTATGTG
Stx2             CAGGGGGCGC GTTCTGTTCG CGCCGTGAAT GAAGAGAGTC AACCAGAATG
Stx2f            CCGGAAGT-T ATTCAGTTCG TTCCGTGAGC CAAAAACAGA AAACAGAATG
Stx2c            CAGGGGGCGC GTTCTGTTCG CGCCGTGAAT GAAGAGAGTC AACCAGAATG
Stx2d-0111       CAGGGGGCGC GTTCCGTTCG CGCCGTGAAT GAAGAGATAC AACCAGAATG
Stx2d-Ount       CAGGGGGCAC GTTCCGTTCG CTCCGTGAAT GAAGAGATAC AACCAGAATG
Stx2v&e          CAGGGCGCGC GTTCTGTTCG CGCCGTGAAT GAAGAGAGTC AACCAGAATG
Stx2era          CAGGGCGCGC GTTCTGTTCG CGCCGTGAAT GAAGAGAGTC AACCAGAATG
Stx2-Ent         CAGGGGGCGC GTTCTGTTCG CGCCGTGAAT GAAGAGAGTC AACCAGAATG


                  ....|....| ....|....| ....|....| ....|....| ....|....|
                       860        870        880        890        900
Stx1             TCCGGCAGAT GGAAGAGTCC GTGGGATTAC GCACAATAAA ATATTGTGGG
Stx-dysenteria   TCCGGCAGAT GGAAGAGTCC GTGGGATTAC GCACAATAAA ATATTGTGGG
Stx-sonnei       TCCGGCAGAT GGAAGAGTCC GTGGGATTAC GCACAATAAA ATATTGTGGG
Stx2             TCAGATAACT GGCGACAGGC CTGTTATAAA AATAAACAAT ACATTATGGG
Stx2f            CCAGATTGTT GGAGACAGGG CGGCCATTAA AGTAAATAAT GTTTTGTGGG
Stx2c            TCAGATAACT GGCGACAGGC CTGTTATAAA AATAAACAAT ACATTATGGG
Stx2d-0111       TCAGATAACT GGCGACAGGC CAGTTATAAG GATAAACAAT ACTTTATGGG
Stx2d-Ount       TCAGATAACT GGCGACAGGC CAGTTATAAG GCTAAACAAT ACTTTATGGG
Stx2v&e          TCAGATAACT GGCGACAGGC CCGTTATAAA AATAAACAAT ACATTATGGG
Stx2era          TCAGATAACT GGCGACAGGC CCGTTATAAA AATAAACAAT ACATTATGGG
Stx2-Ent         TCAGATAACT GGCGACAGGC CCGTTATAAA AATAAACAAT ACATTATGGG


                  ....|....| ....|....| ....|....| ....|....| ....|....|
                       910        920        930        940        950
Stx1             ATTCATCCAC TCTGGGGGCA ATTCTGATGC GCAG-----A ACT-------
Stx-dysenteria   ATTCATCCAC TCTGGGGGCA ATTCTGATGC GCAG-----A ACT-------
Stx-sonnei       ATTCATCCAC TCTGGGGGCA ATTCTGATGC GCAG-----A ACT-------
Stx2             AAAGTAATAC AGCTGCAGCG TTTCTGAACA GAAAGTCACA GTTTTTATAT
Stx2f            AAGCGAATAC AATCGCTGCT TTATTAAATC GCAAGCCTCA GGATCTTACT
Stx2c            AAAGTAATAC AGCTGCAGCG TTTCTGAACA GAAAGTCACA GTTTTTATAT
Stx2d-0111       AAAGTAATAC CGCAGCTGCT TTTCTGAATC GCAGGGCCCA CTCTTTAAAT
Stx2d-Ount       AAAGTAATAC CGCAGCTGCT TTTCTGAATC GCAGGGCTCA CTCTTTAAAT
Stx2v&e          AAAGTAATAC AGCAGCAGCG TTTCTGAACA GAAAGTCACA GTCTTTATAT
Stx2era          AAAGTAATAC AGCAGCAGCG TTTCTGAACA GAAAGTCACA GTCTTTATAT
Stx2-Ent         AAAGTAATAC AGCAGCAGCG TTTCTGAACA GAAAGTCACA GTTTTTATAT


                  ....|....| ....|....| ....|....| ....|....| ....|....|
                       960        970        980        990       1000
Stx1             ---ATTAGCA GTTGA-GGGG GTAAAATGAA AAAAACATTA TTAATAGCTG
Stx-dysenteria   ---ATTAGCA GTTGA-GGGG GTAAAATGAA AAAAACATTA TTAATAGCTG
Stx-sonnei       ---ATTAGCA GTTGA-GGGG GTAAAATGAA AAAAACATTA TTAATAGCTG
Stx2             ACAACGGGTA AATAA-AGGA GTTAAGCATG AAGAAGATGT TTA-TGGCGG
Stx2f            GAACCAAACC AATAACAGGG GGTGAATATG AAGAAGATGA TTA-TTGCAG
Stx2c            ACAACGGGTA AATAA-AGGA GTTAAGCATG AAGAAGATGT TTA-TGGCGG
Stx2d-0111       ACATCCGGAG AATAACAGGA GTTAAATATG AAGAAGATAT TTG-TAGCGG
Stx2d-Ount       ACATCCGGAG AATAACGGGA GTTAAATATG AAGAAGATAT TTG-TAGCGG
Stx2v&e          ACAACTGGTG AATGAAAGGA GTTAAGAATG AAGAAGATGT TTA-TAGCGG
Stx2era          ACAACTGGTG AATGAAAGGA GTTAAGAATG AAGAAGATGT TTA-TAGCGG
Stx2-Ent         ACAACGGGTA AATAA-AGGA GTTAAGTATG AAGAAGATGT TTA-TGGCGG
```

Figure 1-5

```
                ....|....| ....|....| ....|....| ....|....| ....|....|
                  1010       1020       1030       1040       1050
Stx1            CATCGCTTTC ATTTTTTTCA GCAAGTGCGC TGGCGACGCC TGATTGTGTA
Stx-dysenteria  CATCGCTTTC ATTTTTTTCA GCAAGTGCGC TGGCGACGCC TGATTGTGTA
Stx-sonnei      CATCGCTTTC ATTTTTTTCA GCAAGTGCGC TGGCGACGCC TGATTGTGTA
Stx2            TTTTATTTGC ATTAGCTTCT GTTAATGCAA TGGCGGCG-- -GATTGTGCT
Stx2f           TTTTATTTCGG TCTCTTTTCT GCTAATTCCA TGGCGGCG-- -GATTGTGCT
Stx2c           TTTTATTTGC ATTAGTTTCT GTTAATGCAA TGGCGGCG-- -GATTGCGCT
Stx2d-0111      CTTTATTTGC TTTTGTTTCT GTTAATGCAA TGGCAGCT-- -GATTGTGCA
Stx2d-Ount      CTTTATTTGC TTTTGTTTCT GTTAATGCAA TGGCAGCT-- -GATTGTGCA
Stx2v&e         TTTTATTTGC ATTGGTTTCT GTTAATGCAA TGGCGGCG-- -GATTGTGCT
Stx2era         TTTTATTTGC ATTGGTTTCT GTTAATGCAA TGGCGGCG-- -GATTGTGCT
Stx2-Ent        TTTTATTTGC ATTAGTTTCT GTTAATGCAA TGGCGGCG-- -GATTGTGCT


                ....|....| ....|....| ....|....| ....|....| ....|....|
                  1060       1070       1080       1090       1100
Stx1            ACTGGAAAGG TGGAGTATAC AAAATATAAT GATGACGATA CCTTTACAGT
Stx-dysenteria  ACTGGAAAGG TGGAGTATAC AAAATATAAT GATGACGATA CCTTTACAGT
Stx-sonnei      ACTGGAAAGG TGGAGTATAC AAAATATAAT GATGACGATA CCTTTACAGT
Stx2            AAAGGTAAAA TTGAGTTTTC CAAGTATAAT GAGGATGACA CATTTACAGT
Stx2f           GTAGGAAAAA TTGAGTTTTC CAAGTATAAT GAGGATGATA CCTTTACTGT
Stx2c           AAAGGTAAAA TTGAGTTTTC CAAGTATAAT GAGAATGATA CATTCACAGT
Stx2d-0111      AAAGGTAAAA TTGAGTTCTC TAAGTATAAT GAGAATGATA CATTCACAGT
Stx2d-Ount      AAAGGTAAAA TTGAGTTCTC TAAGTATAAT GAGAATGATA CATTCACAGT
Stx2v&e         AAAGGTAAAA TTGAGTTTTC CAAGTATAAT GAGGATAATA CCTTTACTGT
Stx2era         AAAGGTAAAA TTGAGTTTTC CAAGTATAAT GAGGATAATA CCTTTACTGT
Stx2-Ent        AAAGGTAAAA TTGAGTTTTC CAAGTATAAT GAGGATGACA CATTTACAGT


                ....|....| ....|....| ....|....| ....|....| ....|....|
                  1110       1120       1130       1140       1150
Stx1            TAAAGTGGGT GATAAAGAAT TATTTACCAA CAGATGGAAT CTTCAGTCTC
Stx-dysenteria  TAAAGTGGGT GATAAAGAAT TATTTACCAA CAGATGGAAT CTTCAGTCTC
Stx-sonnei      TAAAGTGGGT GATAAAGAAT TATTTACCAA CAGATGGAAT CTTCAGTCTC
Stx2            GAAGGTTGAC GGGAAAGAAT ACTGGACCAG TCGCTGGAAT CTGCAACCGT
Stx2f           GAAGGTGTCA GGAAGAGAAT ACTGGACGAA CAGATGGAAT TTGCAGCCAT
Stx2c           AAAAGTGGCC GGAAAAGAGT ACTGGACCAG TCGCTGGAAT CTGCAACCGT
Stx2d-0111      AAAAGTGGCC GGGAAAGAGT ACTGGACTAA CCGCTGGAAT CTGCAACCGC
Stx2d-Ount      AAAAGTGGCC GGGAAAGAGT ACTGGACTAA CCGCTGGAAT CTGCAACCGC
Stx2v&e         GAAGGTGTCA GGAAGAGAAT ACTGGACGAA CAGATGGAAT TTGCAGCCAT
Stx2era         GAAGGTGTCA GGAAGAGAAT ACTGGACGAA CAGATGGAAT TTGCAGCCAT
Stx2-Ent        GAAGGTTGAC GGGAAAGAAT ACTGGACCAG TCGCTGGAAT CTGCAACCGT


                ....|....| ....|....| ....|....| ....|....| ....|....|
                  1160       1170       1180       1190       1200
Stx1            TTCTTCTCAG TGCGCAAATT ACGGGGATGA CTGTAACCAT TAAAACTAAT
Stx-dysenteria  TTCTTCTCAG TGCGCAAATT ACGGGGATGA CTGTAACCAT TAAAACTAAT
Stx-sonnei      TTCTTCTCAG TGCGCAAATT ACGGGGATGA CTGTAACTAT TAAAACTAAT
Stx2            TACTGCAAAG TGCTCAGTTG ACAGGAATGA CTGTCACAAT CAAATCCAGT
Stx2f           TGTTACAAAG TGCTCAGCTG ACAGGGATGA CTGTAACAAT CATATCTAAT
Stx2c           TACTGCAAAG TGCTCAGTTG ACAGGAATGA CTGTCACAAT CAAATCCAGT
Stx2d-0111      TACTGCAAAG CGCACAGTTA ACAGGAATGA CGGTAACAAT CAAATCAAAT
Stx2d-Ount      TACTGCAAAG CGCACAGTTA ACAGGAATGA CGGTAACAAT CAAATCAAAT
Stx2v&e         TGTTACAAAG TGCTCAGCTG ACAGGGATGA CTGTAACAAT CATATCTAAT
Stx2era         TGTTACAAAG TGCTCAGCTG ACAGGGATGA CTGTAACAAT CATATCTAAT
Stx2-Ent        TACTGCAAAG TGCTCAGTTG ACAGGAATGA CTGTCACAAT CAAATCCAGT
```

Figure 1-6

```
                    ....|....| ....|....| ....|....| ....|....| ....|
                       1210       1220       1230       1240
Stx1            GCCTGTCATA ATGGAGGGGG ATTCAGCGAA GTTATTTTTC GTTGA
Stx-dysenteria  GCCTGTCATA ATGGAGGGGG ATTCAGCGAA GTTATTTTTC GTTGA
Stx-sonnei      GCCTGTCATA ATGGAGGGGG ATTCAGCGAA GTTATTTTTC GTTGA
Stx2            ACCTGTGAAT CAGGCTCCGG ATTTGCTGAA GTGCAGTTTA AT---
Stx2f           ACCTGCAGTT CAGGCTCAGG CTTTGCCCAG GTGAAGTTTA ACTG-
Stx2c           ACCTGTGAAT CAGGCTCCGG ATTTGCTGAA GTGCAGTTTA AT---
Stx2d-0111      ACCTGTGCGT CAGGTTCAGG ATTTGCTGAA GTGCAGTTTA ATT--
Stx2d-Ount      ACCTGTGCGT CAGGTTCAGG ATTTGCTGAA GTGCAGTTTA ATT--
Stx2v&e         ACCTGCAGTT CAGGCTCAGG CTTTGCCCAG GTGAAGTTTA ACTG-
Stx2era         ACCTGCAGTC CAGGCTCAGG CTTTGCCCAG GTGAAGTTTA ACTG-
Stx2-Ent        ACCTGTGAAT CAGGCTCCGG ATTTGCTGAA GTGCAGTTTA AT---
```

Figure 1-7

SEQUENCE LISTING

<110> Cockerill III, Franklin R.
       Rosenblatt, Jon E.
       Sloan, Lynne
       Uhl, James R.

<120> Detection of Shiga Toxin- or Shiga-like
       Toxin-producing Organisms

<130> 07039-397001

<140> US 10/150,792
<141> 2002-05-17

<160> 8

<170> FastSEQ for Windows Version 4.0

<210> 1
<211> 18
<212> DNA
<213> Artificial Sequence
<220>
<223> Oligonucleotide
<400> 1
caagagcgat gttacggt                                                    18

<210> 2
<211> 21
<212> DNA
<213> Artificial Sequence
<220>
<223> Oligonucleotide
<400> 2
aattcttcct acacgaacag a                                                21

<210> 3
<211> 20
<212> DNA
<213> Artificial Sequence
<220>
<223> Oligonucleotide
<400> 3
ctggggaagg ttgagtagcg                                                  20

<210> 4
<211> 21
<212> DNA
<213> Artificial Sequence
<220>
<223> Oligonucleotide
<400> 4
cctgcctgac tatcatggac a                                                21

```
<210> 5
<211> 16
<212> DNA
<213> Artificial Sequence
<220>
<223> Oligonucleotide
<400> 5
gggaccacat cggtgt                                              16

<210> 6
<211> 21
<212> DNA
<213> Artificial Sequence
<220>
<223> Oligonucleotide
<400> 6
cgggcactga tatatgtgta a                                        21

<210> 7
<211> 26
<212> DNA
<213> Artificial Sequence
<220>
<223> Oligonucleotide
<400> 7
ctgtggatat acgagggctt gatgtc                                   26

<210> 8
<211> 22
<212> DNA
<213> Artificial Sequence
<220>
<223> Oligonucleotide
<400> 8
atcaggcgcg ttttgaccat ct                                       22
```